(19) Europäisches Patentamt
European Patent Office
Office européen des brevets

(11) EP 4 570 834 A1

(12) **EUROPEAN PATENT APPLICATION**
published in accordance with Art. 153(4) EPC

(43) Date of publication:
**18.06.2025 Bulletin 2025/25**

(21) Application number: **23852647.9**

(22) Date of filing: **10.08.2023**

(51) International Patent Classification (IPC):
***C08F 220/10*** (2006.01) ***A61K 31/537*** (2006.01)
***A61K 39/395*** (2006.01) ***A61K 45/00*** (2006.01)
***A61K 47/58*** (2017.01) ***A61K 47/64*** (2017.01)
***A61P 35/00*** (2006.01) ***C08F 220/30*** (2006.01)

(52) Cooperative Patent Classification (CPC):
**A61K 31/537; A61K 39/395; A61K 45/00; A61K 47/58; A61K 47/64; A61P 35/00; C08F 220/10; C08F 220/30**

(86) International application number:
**PCT/JP2023/029357**

(87) International publication number:
**WO 2024/034685 (15.02.2024 Gazette 2024/07)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **10.08.2022 JP 2022128537**

(71) Applicant: **KOWA COMPANY, LTD.**
**Naka-ku**
**Nagoya-shi**
**Aichi 460-8625 (JP)**

(72) Inventors:
- **SUZUKI, Kenichi**
  **Higashimurayama-shi, Tokyo 189-0022 (JP)**
- **YOSHIDA, Hideo**
  **Higashimurayama-shi, Tokyo 189-0022 (JP)**
- **FUJIMAKI, Nobuhiro**
  **Higashimurayama-shi, Tokyo 189-0022 (JP)**

(74) Representative: **Wächtershäuser & Hartz Patentanwaltspartnerschaft mbB**
**Weinstraße 8**
**80333 München (DE)**

Remarks:
The complete document including Reference Table(s) and the Sequence Listing(s) can be downloaded from the EPO website

(54) **AFFIBODY MICELLE DRUG CONJUGATE**

(57) A novel copolymer utilizable for drug delivery technology.

The present invention relates to a drug complex in which a target-specific molecule is bonded to a copolymer X comprising structural units of (A), (B), and (C).

EP 4 570 834 A1

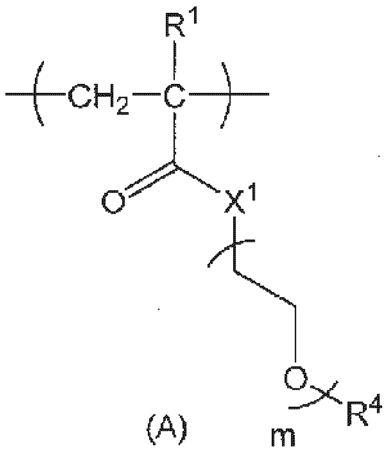

(A) ,

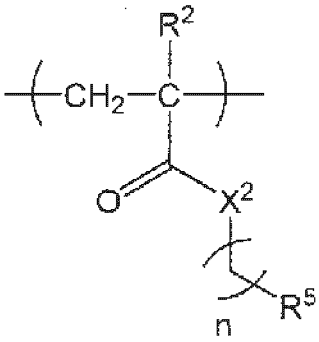

(B) ,

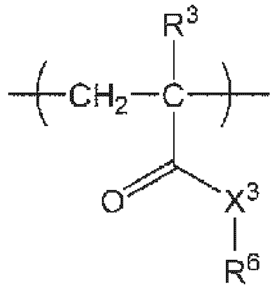

(C)

$R^1$, $R^2$, and $R^3$ are the same or different and represent hydrogen or $C_{1-3}$ alkyl, $R^4$ represents $C_{1-3}$ alkyl, $R^5$ represents hydrogen, $C_{1-18}$ alkyl, 3- to 8- membered cycloalkyl optionally having substituent, adamantyl, $C_{6-18}$ aryl optionally having substituent, or 5- to 10- membered heteroaryl group optionally having substituent, $X^1$, $X^2$, and $X^3$ are the same or different and represent oxygen, sulfur, or N-$R^7$, $R^6$ represents hydrogen, leaving group, or linker, $R^7$ represents hydrogen or $C_{1-3}$ alkyl group, m represents 1 to 100, and n represents 0 to 3.

## Description

The present invention relates to a novel copolymer useful for drug delivery technology. More specifically, the present invention relates to a copolymer for a drug delivery carrier targeting tumors, a pharmaceutical composition in which a physiologically active substance such as an anticancer agent is carried on copolymer, and a medicine containing the composition.

Background Art

In recent years, research on drug delivery systems (DDS) has been energetically conducted as a technique for efficiently and safely delivering drugs to disease sites. Among them, there is an increasing demand for DDS employing nanoparticles as drug delivery carriers as a technique for enhancing the selectivity of drug accumulation by utilizing the structural characteristics of the disease site.

For example, in solid cancer tissue, since the structure of a neovascular vessel (tumor blood vessel) is immature as compared with a normal blood vessel, a cell gap of about several hundred nm is generated in the vascular endothelium, which allows the permeation of a large amount of substance. Due to this structural feature, it is known that a polymeric compound containing nanoparticles selectively permeates a tumor blood vessel and accumulates in solid cancer tissue. Furthermore, in solid cancer tissue, a lymphatic system involved in excretion of polymers malfunctions, so that permeated nanoparticles are continuously retained in the tissue (Enhanced permeability and retention effect, EPR effect). Since a general low molecular drug leaks out of a blood vessel due to membrane permeation of vascular cells, it is non-selectively distributed in tissue and does not accumulate in solid cancer tissue. According to the methodology of the EPR effect, nanoparticle-based drug delivery results in improved tissue selectivity to solid cancer in the distribution of a drug since distribution to tissue is governed by the permeability of vascular endothelial cell gaps. Therefore, the EPR effect is a promising academic support in the development of nanotechnology-applied medicines (nanomedicine) targeting solid cancer.

It is believed that a drug delivery process in the EPR effect occurs through blood flow and that the extravasation process of nanoparticles is passive. Therefore, to maximize the accumulation of nanoparticles in solid cancer, it is important to impart a molecular design that can withstand long-term blood retention to the constituent components of nanoparticles used as drug delivery carriers. Drug delivery carriers are therefore required to have the ability to avoid barriers such as non-specific interactions with blood components, foreign body recognition by the reticuloendothelial system (RES) in the liver, spleen, and lungs, and glomerular filtration in the kidneys. In addition, it is known that these barriers can be overcome by optimizing particle properties such as particle diameter and surface modification with a biocompatible polymer. For example, it is desirable that the particle diameter of the drug delivery carrier be greater than about 6 nm, which is the threshold for renal clearance, and less than 200 nm, which may avoid recognition by the RES.

The particle diameter of the drug delivery carrier is also known to affect tissue permeation at a disease site. For example, the anticancer activity of drug-encapsulating nanoparticles with particle diameters measuring 30 nm, 50 nm, 70 nm, and 100 nm, which exhibit equivalent blood retention, has been compared and studied, and it has been revealed that drug-encapsulating nanoparticles with a particle diameter of 30 nm reach the deep parts of a disease site and thus exhibit the highest therapeutic effect (Non-Patent Literature 1). Therefore, it would be desirable for the particle diameter of nanoparticles for a drug delivery carrier targeting solid cancer to be as small as possible within the range that avoids renal clearance.

As nanoparticles for drug delivery carriers, methods using colloidal dispersions such as liposomes, emulsions, or nanoparticles, methods using biologically derived raw materials such as albumin, methods using natural polymers such as natural polysaccharides, or methods using synthetic polymers, have been developed. Among them, synthetic polymers are widely used as constituents of drug delivery carriers because it is possible to prepare nanoparticles whose particle diameter is precisely controlled by appropriately selecting monomers as constituent components and synthesis methods.

For example, a method for utilizing an amphiphilic block copolymer composed of a hydrophilic segment and a hydrophobic segment as a drug delivery carrier is disclosed. The block copolymer spontaneously associates in an aqueous medium by, for example, hydrophobic interaction between molecules as a driving force to form core-shell type nanoparticles (polymeric micelles). It is known that it is possible to encapsulate a low molecular drug in or bind it to the hydrophobic segment of the polymeric micelle, and the obtained drug-encapsulating polymeric micelle exhibits high blood stability, and due to selective accumulation in solid cancer through the EPR effect, high anticancer activity as compared with the administration of a solution of a low molecular drug (Patent Literature 1). However, since the polymeric micelle is an assembly of multiple molecules, a particle diameter of about 30 nm is the lower limit value that can be prepared, and it is difficult to finely control the size in the vicinity of a particle diameter of 10 nm that can avoid the influence of renal clearance.

Meanwhile, among nanoparticles formed of a synthetic polymer, those which form particles by, for example, chemical cross-linking, hydrophobic interaction, or ionic bonding within a single chain as a driving force (hereinafter abbreviated as single chain nanoparticles (SCNPs)) are known to form nanoparticles having a small particle diameter of 20

nm or less (Non-Patent Literature 2). Therefore, although SCNPs are expected to be useful as drug delivery carriers, a technique for precisely controlling their particle diameter has not been found so far.

Another drug delivery technology is an antibody-drug conjugate (ADC) that allows target delivery of a cytotoxic agent (drug) to antigen-expressing tumor cells (Non-Patent Literatures 3 to 5). The ADC has three components: an antibody (Ab), a linker, and a drug. In order to achieve topical delivery to the target, the drug is linked or conjugated to the antibody. A general conjugation method involves chemical modification via an amino group of a lysine side chain of the antibody or a cysteine sulfhydryl group obtained by reducing an interchain disulfide bond. The design of the ADC remains challenging and a plurality of elements (e.g., selection of antibodies, linker stability, drug/toxin (payload), and cleavage kinetics thereof) must be controlled. Here, an important parameter is the number of payloads to a single antibody (drug-antibody ratio (DAR)). An antibody to which many drugs/toxin molecules are bonded exhibits impaired binding to a target antigen or rapid in vivo clearance from the bloodstream, and generally only a limited number of the drugs/toxin molecules can be bonded to one antibody (typically, the DAR is 4 to 6). As a result, a highly toxic agent with an IC50 of less than 1 nM (e.g., calicheamicin or auristatin monomethyl ester (MMAE)) must be used to obtain efficacy sufficient to kill or damage the target cells (Non-Patent Literatures 6 and 7). Accordingly, if a small part of the conjugate acts on non-target cells, a serious off-target toxicity may appear. In this way, a new approach that combines high efficacy with improved tolerability has been desired.

An affibody molecule, similar to an antibody, is an antibody-mimetic protein capable of specifically binding to a target antigen (Non-Patent Literature 8). Instead of an antibody of the ADC, the drug can be delivered to the target (for example, a tumor cell) without a non-specific interaction, such as Fc receptor binding shown with some antibodies, and with characteristics including a specific target binding ability. A small engineering scaffold protein such as the affibody molecule is promising as a drug carrier.

A complex of the affibody molecule and the drug can be a complement or alternative for current targeting therapies. For example, a human epidermal growth factor receptor 2 (HER2) has been frequently overexpressed in various cancers, and therapies targeting the HER2 have been in routine clinical use. It has been discussed whether a monovalent HER2-binding affibody molecule (ZHER2:2891) can be fused with an albumin-binding domain (ABD) and used as a carrier for a cytotoxic emtansine derivative mcDM1. A monovalent HER2-targeting affibody-drug complex has been reported to have an antitumor activity in vivo (Non-Patent Literature 9). However, the half-life of the affibody-drug complex in blood is very short, and in a case of treatment in which tumor accumulation is enhanced by taking advantage of high target affinity comparable to that of an antibody, an absolute amount of the affibody-drug complex taken into a tumor cannot be obtained, and thus a high dose is required.

Citation List

Patent Literature

Patent Literature 1: JP 3270592 B2

Non-Patent Literature

Non-Patent Literature 1: H. Cabral et al., Nat. Nanotechnol. 6 815-823 (2011)

Non-Patent Literature 2: Jose A. Pomposo, Single-Chain Polymer Nanoparticles: Synthesis, Characterization, Simulations, and Applications (2017)

Non-Patent Literature 3: Chari, R.V. et al., Angew.Chem.Int.Ed. 53, 3796-3827 (2014)

Non-Patent Literature 4: Jagadeesh, D., Smith, M.R., Curr.Treat.Options Oncol. 17, 55 (2016)

Non-Patent Literature 5: Ducry, L., Stump, B., Bioconjugate Chem. 21, 5-13 (2010)

Non-Patent Literature 6: Casi, G., Neri, D., J.Controlled Release 161, 422-428 (2012)

Non-Patent Literature 7: Wu, A. M., Senter, P.D., Nat.Biotechnol. 23, 1137-1146 (2005)

Non-Patent Literature 8: Nord, K., et al. Nature Biotechnol.15, 772-777 (1997)

Non-Patent Literature 9: Xu, Tianqi et al., Cancers 13(1)85 (2020)


Summary of Invention

Technical Problem

It is an object of the present invention to provide a copolymer for a drug delivery carrier targeting a tumor. More specifically, it is an object of the present invention to provide a copolymer for a drug delivery carrier that can improve blood

retention and/or tumor accumulation of a drug.

Solution to Problem

To achieve the above-mentioned objects, the present inventors conducted extensive research and discovered that a terpolymer of an acrylic acid derivative has the property of forming SCNPs in water. Furthermore, the present inventors succeeded in creating a novel polymer for a drug delivery carrier, which is capable of precisely controlling the particle diameter of SCNPs at a minute scale of 20 nm or less and about 10 nm and has high tumor accumulation. When an anticancer agent was carried on or bonded to the polymer and administered to a mouse model with a subcutaneously implanted cancer, it exhibited an excellent antitumor effect. Moreover, a target-specific micelle drug complex was also successfully created.

The present invention relates to the following inventions:

[1] A copolymer in which a target-specific molecule is bonded to a copolymer X comprising structural units represented by the following formulas (A), (B), and (C):

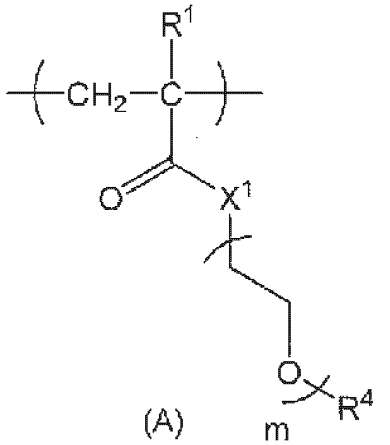
(A) ,
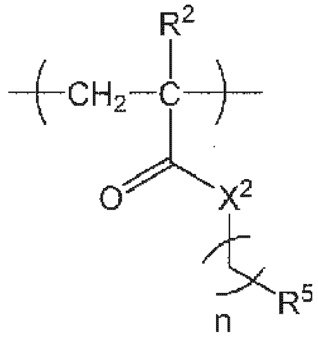
(B) ,
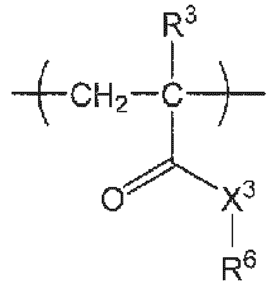
(C)

wherein, $R^1$, $R^2$, and $R^3$ are the same or different and represent a hydrogen atom or a $C_{1-3}$ alkyl group, $R^4$ represents a $C_{1-3}$ alkyl group, $R^5$ represents a hydrogen atom, a $C_{1-18}$ alkyl group, a 3- to 8-membered cycloalkyl group optionally having a substituent, an adamantyl group, a $C_{6-18}$ aryl group optionally having a substituent, or a 5-to 10-membered heteroaryl group optionally having a substituent, $X^1$, $X^2$, and $X^3$ are the same or different and represent an oxygen atom, a sulfur atom, or N-$R^7$, $R^6$ represents a hydrogen atom, a leaving group, or a linker; $R^7$ represents a hydrogen atom or a $C_{1-3}$ alkyl group, m represents an integer of 1 to 100, and n represents an integer of 0 to 3.

[2] The copolymer according to [1], wherein the copolymer X is a copolymer formed by polymerization of three types of monomers represented by the following general formulas (1) to (3):

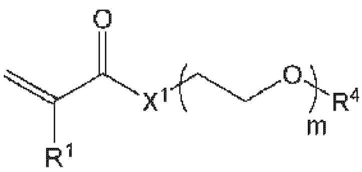
(1) ,
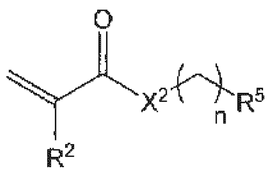
(2) ,
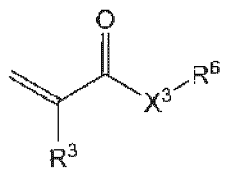
(3)

wherein, $R^1$, $R^2$, and $R^3$ are the same or different and represent a hydrogen atom or a $C_{1-3}$ alkyl group, $R^4$ represents a $C_{1-3}$ alkyl group, $R^5$ represents a hydrogen atom, a $C_{1-18}$ alkyl group, a 3- to 8-membered cycloalkyl group optionally having a substituent, an adamantyl group, a $C_{6-18}$ aryl group optionally having a substituent, or a 5-to 10-membered heteroaryl group optionally having a substituent, $X^1$, $X^2$, and $X^3$ are the same or different and represent an oxygen atom, a sulfur atom, or N-$R^7$, $R^6$ represents a hydrogen atom, a leaving group, or a linker, $R^7$ represents a hydrogen atom or a $C_{1-3}$ alkyl group, m represents an integer of 1 to 100, and n represents an integer of 0 to 3.

[3] The copolymer according to [1] or [2], wherein $R^1$ is a hydrogen atom.

[4] The copolymer according to any one of [1] to [3], wherein $R^2$ is a hydrogen atom.

[5] The copolymer according to any one of [1] to [4], wherein $R^3$ is a hydrogen atom.

[6] The copolymer according to any one of [1] to [5], wherein $R^4$ is a methyl group.
[7] The copolymer according to any one of [1] to [6], wherein $R^5$ is a $C_{6-18}$ aryl group optionally having a substituent.
[8] The copolymer according to any one of [1] to [7], wherein $R^5$ is a phenyl group.
[9] The copolymer according to any one of [1] to [8], wherein $R^6$ is a hydrogen atom.
[10] The copolymer according to any one of [1] to [8], wherein the leaving group of $R^6$ is a group represented by the following formula (4).

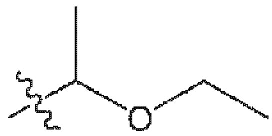

(4)

[11] The copolymer according to any one of [1] to [8], wherein the linker of $R^6$ is represented by the following formula (5):

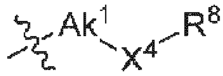

(5)

wherein, $R^8$ represents a hydrogen atom or a drug, $Ak^1$ represents a $C_{1-7}$ alkylene bond, and $X^4$ represents an oxygen atom, a sulfur atom, or -N($R^7$)- ($R^7$ represents a hydrogen atom or a $C_{1-3}$ alkyl group).
[12] The copolymer according to any one of [1] to [11], wherein $X^1$ is an oxygen atom.
[13] The copolymer according to any one of [1] to [12], wherein $X^2$ is an oxygen atom.
[14] The copolymer according to any one of [1] to [13], wherein $X^3$ is an oxygen atom or NH.
[15] The copolymer according to any one of [1] to [14], wherein m is an integer of 4 to 22.
[16] The copolymer according to any one of [1] to [15], wherein n is 1.
[17] The copolymer according to any one of [1] to [16], wherein a ratio of structural units (A), (B), and (C) is composed of 0.01 to 100 parts by mass of (B) and 0.1 to 100 parts by mass of (C) with respect to 1 part by mass of (A).
[18] The copolymer according to any one of [2] to [16], wherein 0.01 to 100 parts by mass of monomer (2) and 0.1 to 100 parts by mass of monomer (3) are polymerized with respect to 1 part by mass of monomer (1).
[19] The copolymer according to any one of [1] to [18], wherein the copolymer has a number average molecular weight of 5 000 to 150 000.
[20] The copolymer according to any one of [1] to [19], wherein the target-specific molecule is an affibody.
[21] The copolymer according to any one of [1] to [19], wherein the affibody is an anti-HER2 affibody, an anti-HER3 affibody, an anti-EGFR affibody, an anti-PD-L1 affibody, an anti-TNF$\alpha$ affibody, an anti-fibrinogen affibody, an anti-transferrin affibody, an anti-HSA affibody, an anti-insulin affibody, an anti-IgG affibody, an anti-IgM affibody, an anti-IgA affibody, or an anti-IgE affibody.
[22] The copolymer according to any one of [1] to [19], wherein the affibody is a molecule including an amino acid sequence selected from the following amino acid sequences (2) to (8):

AEAKYAKEMRNAYWEIALLPNLTNQQKRAFIRKLYDDPSQSSELLSEAKKLNDSQAPK (SEQ ID (2)) (2)
AEAKYAKEKYNAYYEIWQLPNLTKYQKAAFIGKLQDDPSQSSELLSEAKKLNDSQAPK (SEQ ID (3)) (3)
VDNKFNKEMWAAWEEIRNLPNLNGWQMTAFIASLVDDPSQSANLLAEAKKLNDAQAPK (SEQ ID (4)) (4)
VDNKFNKEMWIAWEEIRDLPNLNGWQMTAFIASLLDDPSQSANLLAEAKKLNDAQAPK (SEQ ID (5)) (5)
AEAKYAKEMWIAWEEIRNLPNLNGWQMTAFIAKLLDDPSQSSELLSEAKKLNDSQAPK (SEQ ID (6)) (6)
AEAKYAKERNAAAYEILYLPNLTNAQKWAFIWKLDDDPSQSSELLSEAKKLNDSQAPK (SEQ ID (7)) (7)
AEAKYAKERNKAAYEILYLPNLTNAQKWAFIWKLDDDPSQSSELLSEAKKLNDSQAPK (SEQ ID (8))

(8)

[23] A drug complex including the copolymer according to any one of [1] to [22] and a drug.
[24] The drug complex according to [23], wherein the drug is an antimetabolite, an alkylating agent, an anthracycline, an antibiotic, a mitotic inhibitor, a topoisomerase inhibitor, a proteasome inhibitor, or an anti-hormone agent.
[25] The drug complex according to [23], wherein the drug is DM0, DM1, DM2, DM3, DM4, emtansine, auristatin E, auristatin phenylalanine phenylenediamine (AFP), monomethyl auristatin E, monomethyl auristatin D, monomethyl auristatin F, paclitaxel, docetaxel, irinotecan, topotecan, nogitecan, amsacrine, etoposide, teniposide, mizantrone, SN-38, exatecan, or deruxtecan.
[26] The drug complex according to any one of [23] to [25], wherein the bond of the target-specific molecule or the drug to the copolymer X is a covalent bond or a noncovalent bond.
[27] A single chain nanoparticle including the copolymer or the drug complex according to any one of [1] to [26].
[28] A pharmaceutical composition including the copolymer or the drug complex according to any one of [1] to [27].

Advantageous Effects of Invention

As will be apparent from examples described later, the SCNP obtained by self-association of the copolymer of the present invention, which is carried with or bonded to an anticancer agent, exhibited an effect of suppressing tumor growth in a mouse cancer-carrying model, and thus can be applied as a therapeutic agent for malignant tumors. The SCNP obtained by the self-association of the copolymer of the present invention, which is carried with or bonded to the anticancer agent, makes it possible to obtain a high DAR as compared with an existing ADC, and has a high effect of suppressing the tumor growth at a low dose, so that it is possible to provide a therapeutic agent for malignant tumors capable of achieving both pharmacological action enhancement and side effect suppression. Then, the copolymer to which the target-specific molecule of the present invention is bonded is useful as a drug delivery utilizing a molecule that specifically recognizes the target.

Brief Description of Drawings

Fig. 1 is a diagram showing a $^1$H-NMR spectrum of a copolymer obtained in Example 1, measured by using nuclear magnetic resonance (NMR).
Fig. 2 is a diagram showing a chromatogram of the copolymer obtained in Example 1 obtained by gel permeation chromatography (GPC).
Fig. 3 is a diagram showing a particle diameter measurement result (scattering intensity distribution) in dynamic light scattering (DLS) for a copolymer before encapsulating DACHPt (Example 69) and a DACHPt-encapsulating SCNP (Example 70).
Fig. 4 is a diagram showing a $^1$H-NMR spectrum of $N_3$-poly[(benzyl acrylate)-co-(poly(ethylene glycol)methyl ether acrylate)-co-(acrylic acid)] obtained in Example 72, measured by using the nuclear magnetic resonance (NMR).
Fig. 5 is a diagram showing a chromatogram obtained by the gel permeation chromatography (GPC) for $N_3$-poly [(benzyl acrylate)-co-(poly(ethylene glycol)methyl ether acrylate)-co-(acrylic acid)] obtained in Example 72.
Fig. 6 is a diagram showing a $^1$H-NMR spectrum of a 1,4-diaminobutane-bonded copolymer obtained in Example 73, measured by using the nuclear magnetic resonance (NMR).
Fig. 7 is a diagram showing a $^1$H-NMR spectrum of a DM1-SPDP-1,4-diaminobutane-bonded copolymer obtained in Example 74, measured by using the nuclear magnetic resonance (NMR).
Fig. 8 is a diagram showing a particle diameter measurement result (scattering intensity distribution) of the anti-HER2 affibody micelle drug complex obtained in Example 75 in dynamic light scattering (DLS).
Fig. 9 is a diagram showing a particle diameter measurement result (scattering intensity distribution) of the anti-EGFR affibody micelle drug complex obtained in Example 76 in dynamic light scattering (DLS).
Fig. 10 is a diagram showing a particle diameter measurement result (scattering intensity distribution) of an anti-HER3 affibody micelle drug complex obtained in Example 77 in dynamic light scattering (DLS).
Fig. 11 is a diagram showing a change in relative tumor volume when an oxaliplatin solution or the DACHPt-encapsulating SCNP (Example 70) was administered 3 times every other day to a model mouse having a back subcutaneously transplanted with a mouse colon cancer cell line (C26).

Description of Embodiments

Terms in the present description are used in the meanings commonly used in the field unless otherwise specified.

Hereinafter, the present invention will be described in more detail.

In the present description, the term "nanoparticle" refers to a structure showing a particle diameter of 100 nm or less.

In the present description, the term "single chain nanoparticle (SCNP)" refers to a nanoparticle formed by using, for example, chemical cross-linking, hydrophobic interaction, or ionic bonding in a single chain as a driving force. The SCNP often indicates a nanoparticle having a relatively small particle diameter of 20 nm or less among nanoparticles.

In the present description, the term "initiator" means an initiator for thermal radical polymerization such as an azo compound or a peroxide.

In the present specification, the term "chain transfer agent" refers to a compound that causes a chain transfer reaction in radical polymerization, and is preferably a compound having a thiocarbonyl group.

In the present description, the term "$C_{1-3}$ alkyl group" means a linear or branched, alkyl group having 1 to 3 carbon atoms, and examples thereof include a methyl group, an ethyl group, an n-propyl group, and an isopropyl group.

In the present description, the term "$C_{1-18}$ alkyl group" means a linear or branched, alkyl group having 1 to 18 carbon atoms, and examples thereof include a methyl group, an ethyl group, an n-propyl group, an isopropyl group, an n-butyl group, an isobutyl group, a sec-butyl group, a tert-butyl group, a pentyl group, a hexyl group, a heptyl group, an octyl group, a nonyl group, a decyl group, an undecyl group, a dodecyl group, a tridecyl group, a tetradecyl group, a pentadecyl group, a hexadecyl group, a heptadecyl group, and an octadecyl group.

In the present description, the term "3- to 8-membered cycloalkyl group optionally having a substituent" means a cyclic alkyl group having 3 to 8 carbon atoms, and examples thereof include a cyclopropyl group, a cyclobutyl group, a cyclopentyl group, a cyclohexyl group, a cycloheptyl group, and a cyclooctyl group. The substituent is not particularly limited, and examples thereof include a halogen atom, an alkyl group having 1 to 6 carbon atoms, an alkenyl group having 2 to 6 carbon atoms, an alkynyl group having 2 to 6 carbon atoms, a hydroxyl group, an alkoxy group having 1 to 6 carbon atoms, an amino group, an alkylamino group having 1 to 6 carbon atoms, a di-alkylamino group having 1 to 6 carbon atoms in which alkyl groups are the same or different, a thiol group, an alkylthio group having 1 to 6 carbon atoms, a carboxyl group, an alkoxycarbonyl group having 1 to 6 carbon atoms, and a carbamoyl group.

In the present description, the term "$C_{6-18}$ aryl group optionally having a substituent" means a monocyclic or polycyclic condensed aromatic hydrocarbon group, and examples thereof include a phenyl group, a naphthyl group, an anthracenyl group, a phenanthrenyl group, a triphenylenyl group, a pyrenyl group, a chrysenyl group, and a naphthacenyl group. Further, a "$C_{6-14}$ aryl group optionally having a substituent" means a monocyclic or polycyclic condensed aromatic hydrocarbon group, and examples thereof include a phenyl group, a naphthyl group, an anthracenyl group, and a phenanthrenyl group. The substituent is not particularly limited, and examples thereof include a halogen atom, an alkyl group having 1 to 6 carbon atoms, an alkenyl group having 2 to 6 carbon atoms, an alkynyl group having 2 to 6 carbon atoms, a hydroxyl group, an alkoxy group having 1 to 6 carbon atoms, an amino group, an alkylamino group having 1 to 6 carbon atoms, a di-alkylamino group having 1 to 6 carbon atoms in which alkyl groups are the same or different, a thiol group, an alkylthio group having 1 to 6 carbon atoms, a carboxyl group, an alkoxycarbonyl group having 1 to 6 carbon atoms, and a carbamoyl group.

In the present description, the term "5- to 10-membered heteroaryl group optionally having a substituent" means a 5- to 10-membered, monocyclic aromatic heterocyclic group or condensed aromatic heterocyclic group containing 1 to 4 heteroatoms selected from a nitrogen atom, an oxygen atom, and a sulfur atom, other than a carbon atom, as atoms constituting the ring. Examples of the monocyclic aromatic heterocyclic group include a furyl group, a thienyl group, a pyrrolyl group, a pyridinyl group, a pyrazinyl group, a pyrimidinyl group, a pyridazinyl group, an imidazolyl group, a pyrazyl group, a thiallyl group, an oxazolyl group, an isoxazolyl group, a 1,3,4-thiadiazolyl group, a 1,2,3-triazolyl group, a 1,2,4-triazolyl group, and a tetrazolyl group. Examples of the condensed aromatic heterocyclic group include a benzofuranyl group, a benzothiophenyl group, a quinoxalinyl group, an indolyl group, an isoindolyl group, an isobenzofuranyl group, a chromanyl group, a benzimidazolyl group, a benzothiazolyl group, a benzoxazolyl group, a quinolyl group, and an isoquinolinyl group. The term "6- to 10-membered heteroaryl group optionally having a substituent" means a 6- to 10-membered, monocyclic aromatic heterocyclic group or condensed aromatic heterocyclic group containing 1 to 4 heteroatoms selected from a nitrogen atom, an oxygen atom, and a sulfur atom, other than a carbon atom, as the atoms constituting the ring. Examples of the monocyclic aromatic heterocyclic group include a pyridinyl group, a pyrazinyl group, a pyrimidinyl group, and a pyridazinyl group. Examples of the condensed aromatic heterocyclic group include a benzofuranyl group, a benzothiophenyl group, a quinoxalinyl group, an indolyl group, an isoindolyl group, an isobenzofuranyl group, a chromanyl group, a benzimidazolyl group, a benzothiazolyl group, a benzoxazolyl group, a quinolyl group, and an isoquinolinyl group. The substituent is not particularly limited, and examples thereof include a halogen atom, an alkyl group having 1 to 6 carbon atoms, an alkenyl group having 2 to 6 carbon atoms, an alkynyl group having 2 to 6 carbon atoms, a hydroxyl group, an alkoxy group having 1 to 6 carbon atoms, an amino group, an alkylamino group having 1 to 6 carbon atoms, a di-alkylamino group having 1 to 6 carbon atoms in which alkyl groups are the same or different, a thiol group, an alkylthio group having 1 to 6 carbon atoms, a carboxyl group, an alkoxycarbonyl group having 1 to 6 carbon atoms, and a carbamoyl group.

In the present description, examples of the "halogen atom" include a fluorine atom, a chlorine atom, a bromine atom, and an iodine atom.

In the present description, the term "$C_{1-7}$ alkylene bond" means a linear or branched, alkylene group having 1 to 7 carbon atoms, which may be substituted, and examples thereof include a methylene group, an ethylene group, a propylene group, a butylene group, a pentylene group, a hexylene group, a heptylene group, $-CH(CH_3)-$, $-C(CH_3)_2-$, $-CH(CH_2CH_3)-$, $-CH(CH_3)CH_2-$, $-CH(CH_2CH_2CH_3)-$, $-CH(CH_2(CH_3)_2)$ $-C(CH_3)(CH_2CH_3)-$, $-C(CH_3)_2CH_2-$, $-CH(CH_2CH_3)CH_2$, $-CH(CH_3)CH(CH_3)-$, $-CH(CH_3)CH_2CH_2-$, $-CH_2CH(CH_3)CH_2-$, $-CH(CH_2CH_2CH_2CH_3)-$, $-C(CH_3)(CH_2CH_2CH_3)-$, $-C(CH_2CH_3)_2-$, $-CH(CH_2CH_2CH_3)CH_2-$, $-CH(CH_2(CH_3)_2)CH_2-$, $-C(CH_3)(CH_2CH_3)CH_2-$, $-C(CH_3)_2CH(CH_3)-$, $-CH(CH_2CH_3)CH(CH_3)-$, $-C(CH_3)_2CH_2CH_2-$, $-CH(CH_2CH_3)CH_2CH_2-$, $-CH_2CH(CH_2CH_3)CH_2-$, $-CH(CH_3)CH(CH_3)CH_2-$, $-CH(CH_3)CH_2CH(CH_3)-$, $-CH(CH_2CH_2CH_2CH_2CH_3)-$, $-C(CH_3)(CH_2CH_2CH_2CH_3)-$, $-C(CH_2CH_3)(CH_2CH_2CH_3)-$, $-C(CH_2CH_3)(CH_2(CH_3)_2)-$, $-CH(CH_2CH_2CH_2CH_3)CH_2-$, $-CH(CH(CH_3)CH_2CH_3)CH_2-$, $-CH(CH_2CH(CH_3)CH_3)CH_2-$, $-CH(CH_2CH_2(CH_3)_2)CH_2-$, $-C(CH_3)(CH_2CH_2CH_3)CH_2-$, $-C(CH_2CH_3)_2CH_2-$, $-CH(CH_2(CH_3)_2)CH_2-$, $-C(CH_3)(CH_2CH_3)CH(CH_3)-$, $-CH(CH_2CH_3)C(CH_3)_2-$, $-CH(CH_2CH_3)CH(CH_2CH_3)-$, $-C(CH_3)_2C(CH_3)_2-$, $-CH(CH_2CH_2CH_3)CH_2CH_2-$, $-CH(CH_2(CH_3)_2)CH_2CH_2-$, $-CH_2CH(CH_2CH_2CH_3)CH_2-$, $-CH_2CH(CH_2(CH_3)_2)CH_2-$, $-C(CH_3)(CH_2CH_3)CH_2CH_2-$, $-CH(CH_2CH_3)CH(CH_3)CH_2-$, $-CH(CH_2CH_3)CH_2CH(CH_3)-$, $-CH_2CH(CH_2CH_3)CH(CH_3)-$, $-CH(CH_3)CH(CH_3)CH(CH_3)-$, $-C(CH_3)_2CH(CH_3)CH_2-$, $-C(CH_3)_2CH_2CH(CH_3)-$, $-CH(CH_2CH_3)CH_2CH_2CH_2-$, $-CH_2CH(CH_2CH_3)CH_2CH_2-$, $-C(CH_3)_2CH_2CH_2CH_2-$, $-CH(CH_3)CH(CH_3)CH_2CH_2-$, $-CH(CH_3)CH_2CH(CH_3)CH_2-$, $-CH(CH_3)CH_2CH_2CH(CH_3)-$, $-CH(CH_3)CH_2CH_2CH_2CH_2-$, $-CH_2CH(CH_3)CH_2CH_2CH_2-$, $-CH_2CH_2CH(CH_3)CH_2CH_2-$, $-C(CH_2CH_3)(CH_2CH_2CH_2CH_3)-$, $-C(CH_2CH_2CH_3)_2-$, $-C(CH_3)(CH_2CH_2CH_2CH_3)CH_2-$, $-C(CH_3)(CH(CH_3)CH_2CH_3)CH_2-$, $-C(CH_3)(CH_2CH(CH_3)CH_3)CH_2-$, $-C(CH_3)(CH_2CH_2(CH_3)_2)CH_2-$, $-CH(CH_2CH_2CH_2CH_3)CH(CH_3)-$, $-CH(CH(CH_3)CH_2CH_3)CH(CH_3)-$, $-CH(CH_2CH(CH_3)CH_3)CH(CH_3)-$, $-CH(CH_2CH_2(CH_3)_2)CH(CH_3)-$, $-C(CH_3)(CH_2CH_2CH_3)CH(CH_3)-$, $-C(CH_2CH_3)_2CH(CH_3)-$, $-CH(CH_2(CH_3)_2)CH(CH_3)-$, $-C(CH_3)(CH_2CH_3)C(CH_3)_2-$, $-C(CH_3)(CH_2CH_2CH_3)CH_2CH_2-$, $-CH(CH_2CH_2CH_3)CH(CH_3)CH_2-$, $-CH(CH_2CH_2CH_3)CH_2CH(CH_3)-$, $-CH_2CH(CH_2CH_2CH_3)CH(CH_3)-$, $-C(CH_3)(CH_2CH_3)CH(CH_3)CH_2-$, $-C(CH_3)(CH_2CH_3)CH_2CH(CH_3)-$, $-CH(CH_2CH_3)C(CH_3)_2CH_2-$, $-CH(CH_2CH_3)CH(CH_3)CH(CH_3)-$, $-CH(CH_2CH_3)CHCH(CH_3)_2-$, $-C(CH_3)_2CH(CH_2CH_3)CH_2-$, $-CH(CH_3)C(CH_3)(CH_2CH_3)CH_2-$, $-CH(CH_3)CH(CH_2CH_3)CH(CH_3)-$, $-C(CH_3)_2CH(CH_3)CH(CH_3)-$, $-CH(CH_3)C(CH_3)_2CH(CH_3)-$, $-C(CH_3)_2CH(CH_3)CH_2CH_2-$, $-C(CH_3)_2CH_2CH(CH_3)CH_2-$, $-C(CH_3)_{2C}H_2CH_2CH(CH_3)-$, $-CH(CH_3)C(CH_3)_2CH_2CH_2-$, $-CH(CH_3)CH(CH_3)CH(CH_3)CH_2-$, $-CH(CH_3)CH(CH_3)CH_2CH(CH_3)-$, $-CH(CH_3)CH(CH_3)CH_2CH_2CH_2-$, $-CH(CH_3)CH_2CH(CH_3)CH_2CH_2-$, $-CH(CH_3)CH_2CH_2CH(CH_3)CH_2-$, $-CH(CH_3)CH_2CH_2CH_2CH(CH_3)-$, $-C(CH_3)_2CH_2CH_2CH_2CH_2-$, and $-CH(CH_2CH_3)CH_2CH_2CH_2CH_2$. The substituent is not particularly limited, and can substitute any hydrogen atom, and examples thereof include a phenoxy group, a carboxylic acid, a sulfonic acid, a phosphoric acid, a hydroxyl group, and a thiol group.

In the present description, the "target-specific molecule" is a molecule that recognizes and specifically binds to a marker or a receptor (for example, a transmembrane protein, a surface insolubilized protein, or a proteoglycan) expressed on a cell surface. The target-specific molecule is a molecule that specifically recognizes a target specific to or overexpressed in cancer cells necessary for cancer growth or metastasis, and is a peptide or a peptidomimetic. Examples of the peptide or the peptidomimetic include an affibody molecule (for example, ABY-025), an integrin-targeting peptide (RGD peptide), an LHRH receptor-targeting peptide, an ErbB2 (HER2) receptor-targeting peptide, a prostate-specific membrane binding antigen (PSMA)-targeting peptide, a lipoprotein receptor LRP1-targeting peptide, an ApoE protein-derived peptide, an ApoA protein peptide, a somatostatin receptor-targeting peptide, a chlorotoxin-derived peptide, bombesin, insulin, transferrin, a fibrinogen-gamma fragment, thrombospondin, claudin, an ankyrin repeat protein, an ankyrin-like repeat protein, and a synthetic peptide. Hereinafter, the copolymer X of the present invention bonded to or carried with the target-specific molecule may be referred to as a "target-specific copolymer".

In the present description, the "affibody" is a polypeptide capable of specifically binding to a target antigen. The affibody is a B domain derived from an IgG-binding domain of a Staphylococcus protein A (protein A produced by Staphylococcus aureus), with three $\alpha$-helix structures. Further, there is a Z domain as a synthetic IgG-binding domain.

A wild-type sequence includes a 58-amino acid sequence represented by the following amino acid SEQ ID (1).
VDNKFNKEQQNAFYEILHLPNLNEEQRNAFIQSLKDDPSQSANLLAEAKKLNDAQAPK (1)

The affibody molecule has a sequence in which one or more amino acids are deleted, substituted, inserted, or added synchronously or differently to enhance specific affinity for the target. Preferably, any amino acid can be substituted at positions 9, 10, 11, 13, 14, 17, 18, 24, 25, 27, 28, 32, and 35. Molecules that specifically bind to a target antigen (for example, HER-2 or EGFR) can be selected from a dedicated affibody library (for example, US 5,831,012) by a screening method known in the art. The affibody molecule may be a monomer, or the same or different target-specific affibody molecules may also be used in combination. Different targets include, for example, an albumin-binding affibody as described in WO 2009/016043 A. Further, the affibody molecule can also be used in conjugation with a weak electrophile that covalently binds to the target.

Characteristics of the affibody molecule include high solubility, tissue penetration, stability to heat and enzymes, and relatively low production cost.

The affibody molecule may be carried on the copolymer by an action such as electrostatic interaction, hydrogen

bond, hydrophobic interaction, or a covalent bond to the copolymer X of the present invention. The affibody molecule can be modified to an extent that the binding specificity is maintained and can bind to the copolymer X via the head (position 1) or the tail (position 58).

In the present description, the term "pharmaceutical composition" means one in which active ingredients (drug, physiologically active substance) that can be used for diagnosis, prevention, or treatment of a disease are carried on the copolymer X, the target-specific copolymer, or the drug complex of the present invention by an action such as electrostatic interaction, hydrogen bond, hydrophobic interaction, or covalent bonding. Examples of the carrying form include a form in which the drug is present on the particle surface, a form in which the drug is contained in the nanoparticle, and a combination form thereof where the target-specific copolymer or the drug complex forms the nanoparticle.

One embodiment of the present invention is a copolymer or a drug complex in which a target-specific molecule is bonded to a copolymer X comprising structural units represented by the following formulas (A), (B), and (C).

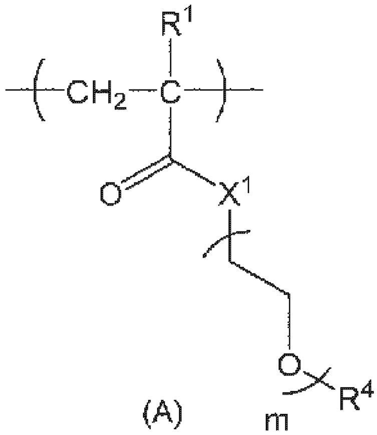

(A) ,

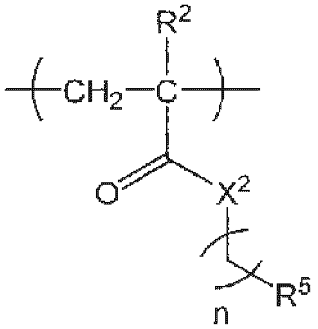

(B) ,

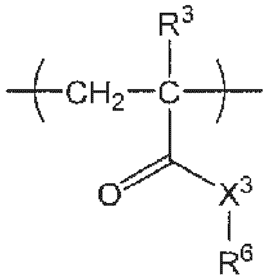

(C)

wherein, $R^1$, $R^2$, and $R^3$ are the same or different and represent a hydrogen atom or a $C_{1-3}$ alkyl group, $R^4$ represents a $C_{1-3}$ alkyl group, $R^5$ represents a hydrogen atom, a $C_{1-18}$ alkyl group, a 3- to 8-membered cycloalkyl group optionally having a substituent, an adamantyl group, a $C_{6-18}$ aryl group optionally having a substituent, or a 5-to 10-membered heteroaryl group optionally having a substituent, $X^1$, $X^2$, and $X^3$ are the same or different and represent an oxygen atom, a sulfur atom, or N-$R^7$, $R^6$ represents a hydrogen atom, a leaving group, or a linker, $R^7$ represents a hydrogen atom or a $C_{1-3}$ alkyl group, m represents an integer of 1 to 100, and n represents an integer of 0 to 3.

In the copolymer X of the present invention, the structural unit (A) functions as a unit that imparts hydrophilicity, and the structural unit (B) functions as a unit that imparts hydrophobicity. Further, the structural unit (C) functions as a scaffold in which the active ingredient (drug, or physiologically active substance) is bonded to the copolymer X or the target-specific copolymer. Having these three structural units serves to imparting the copolymer X, the target-specific copolymer, or the drug complex of the present invention a property of forming SCNP in water, and to facilitating the formed SCNP to precisely control particle diameter at a minute scale of 20 nm or less, and to function as a drug delivery carrier having high tumor accumulation.

$R^1$ in the structural unit (A) represents a hydrogen atom or a $C_{1-3}$ alkyl group, and is preferably a hydrogen atom or a methyl group, preferably a hydrogen atom, an ethyl group, or a propyl group, and more preferably a hydrogen atom.

$X^1$ represents an oxygen atom, the sulfur atom, or N-$R^7$, and is preferably an oxygen atom, a sulfur atom, or NH, and more preferably an oxygen atom.

m represents an integer of 1 to 100, is preferably an integer of 3 to 100, and from a viewpoint of imparting good hydrophilicity, preferably 3 to 80, more preferably 4 to 60, still more preferably 4 to 40, and yet more preferably 4 to 22.

$R^4$ represents a $C_{1-3}$ alkyl group, specifically a methyl group, an ethyl group, an n-propyl group or an isopropyl group, preferably a methyl group or an ethyl group, and more preferably a methyl group.

$R^2$ in the structural unit (B) represents a hydrogen atom or a $C_{1-3}$ alkyl group, and is preferably a hydrogen atom or a methyl group, preferably a hydrogen atom, an ethyl group, or a propyl group, and more preferably a hydrogen atom.

$X^2$ represents an oxygen atom, a sulfur atom, or N-$R^7$, and is preferably an oxygen atom, a sulfur atom, or NH, and more preferably an oxygen atom.

n represents an integer of 0 to 3, preferably an integer of 1 to 3, and more preferably 1.

$R^5$ represents a hydrogen atom, a $C_{1-18}$ alkyl group, a 3- to 8-membered cycloalkyl group optionally having a substituent, an adamantyl group, a $C_{6-18}$ aryl group optionally having a substituent, or a 5- to 10-membered heteroaryl group optionally having a substituent, and from a viewpoint of imparting the hydrophobicity to the structural unit (B), preferably a $C_{1-18}$ alkyl group, a 3- to 8-membered cycloalkyl group optionally having a substituent, an adamantyl group, a $C_{6-18}$ aryl group optionally having a substituent, or a 5- to 10-membered heteroaryl group optionally having a substituent,

more preferably a $C_{1-18}$ alkyl group, a 3- to 8-membered cycloalkyl group optionally having a substituent, an adamantyl group, a $C_{6-18}$ aryl group optionally having a substituent, or a 5- to 10-membered heteroaryl group optionally having a substituent, and still more preferably a $C_{1-18}$ alkyl group, a 3- to 8-membered cycloalkyl group, an adamantyl group, or a $C_{6-18}$ aryl group. Meanwhile, a 3- to 8-membered cycloalkyl group optionally having a substituent, an adamantyl group, a $C_{6-14}$ aryl group optionally having a substituent, or a 6- to 10-membered heteroaryl group optionally having a substituent is also preferable. Here, the substituent is preferably one or more types selected from a halogen atom, an alkyl group having 1 to 6 carbon atoms, an alkenyl group having 2 to 6 carbon atoms, and an alkynyl group having 2 to 6 carbon atoms.

$R^3$ in the structural unit (C) represents a hydrogen atom or a $C_{1-3}$ alkyl group, and is preferably a hydrogen atom or a methyl group, preferably a hydrogen atom, an ethyl group, or a propyl group, and more preferably a hydrogen atom.

$X^3$ represents an oxygen atom, a sulfur atom, or N-$R^7$, and is preferably an oxygen atom, a sulfur atom, or NH, and more preferably an oxygen atom or NH.

$R^6$ represents a hydrogen atom, a leaving group, or a linker. The leaving group is a group that can detach when the structural unit (C) binds to the drug (physiologically active substance), and the linker is a group that can be used for crosslinking when the structural unit (C) binds to the drug (physiologically active substance). As the leaving group or linker, a $C_{1-18}$ alkyl group optionally having a substituent, a 3- to 8-membered cycloalkyl group optionally having a substituent, or a $C_{7-19}$ aralkyl group optionally having a substituent is preferable. Here, examples of the substituent include a halogen atom, an alkyl group having 1 to 6 carbon atoms, an alkenyl group having 2 to 6 carbon atoms, an alkynyl group having 2 to 6 carbon atoms, a hydroxyl group, an alkoxy group having 1 to 6 carbon atoms, an amino group, an alkylamino group having 1 to 6 carbon atoms, a di-alkylamino group having 1 to 6 carbon atoms in which alkyl groups are the same or different, a thiol group, an alkylthio group having 1 to 6 carbon atoms, a carboxyl group, an alkoxycarbonyl group having 1 to 6 carbon atoms, and a carbamoyl group. Among these groups, the linker is preferably a group having a functional group such as a hydroxyl group, an amino group, a thiol group, or a carboxyl group as a substituent.

Preferred specific examples of the leaving group of $R^6$ include a group represented by the following formula (4) :

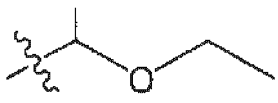

(4)

Preferred specific examples of the linker of $R^6$ include a group represented by the following formula (5):

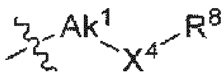

(5)

wherein, $R^8$ represents a hydrogen atom or a drug, $Ak^1$ represents a $C_{1-7}$ alkylene bond, and $X^4$ represents an oxygen atom, a sulfur atom, or -N($R^7$)- ($R^7$ represents a hydrogen atom or a $C_{1-3}$ alkyl group). In the formula (5), $X^4$ more preferably represents an oxygen atom, a sulfur atom, or NH.

More preferred examples of the linker of $R^6$ include groups selected from the following formulas (6) to (8).

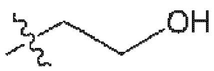

(6)

,

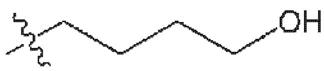

(7)

,

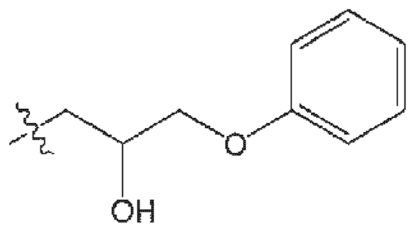

(8)

The copolymer X of the present invention is the copolymer having the structural units represented by formulas (A), (B) and (C). The copolymer X may be a random copolymer or a block copolymer, and is preferably a random copolymer. As for a composition ratio of each structural unit in one molecule, when (A) is 1 part by mass, preferably (B) is 0.01 to 100 parts by mass and (C) is 0.1 to 100 parts by mass; more preferably (B) is 0.05 to 18 parts by mass and (C) is 0.1 to 20 parts by mass, and particularly preferably (B) is 0.05 to 4 parts by mass and (C) is 0.1 to 16 parts by mass.

A polymerization degree of the copolymer X of the present invention is not particularly limited, and it is preferably 5 000 to 150 000, and more preferably 8 000 to 150 000 as a number average molecular weight.

In the copolymer of the present invention, as described above, the monomer represented by general formula (1) functions as a unit that imparts the hydrophilicity, and the monomer represented by general formula (2) functions as a unit that imparts the hydrophobicity. Further, the monomer represented by general formula (3) functions as a scaffold to which the drug and the copolymer are bonded. Examples of the monomer functioning as the hydrophobic unit represented by general formula (2) include monomers represented by the following formulas.

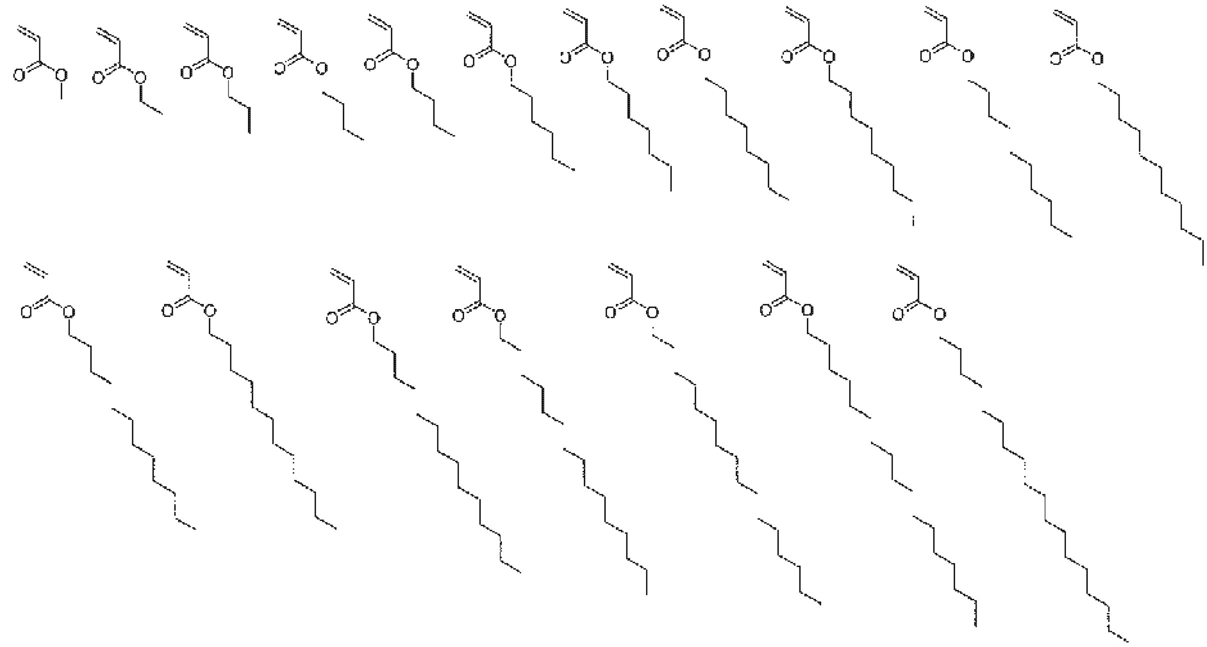

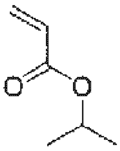 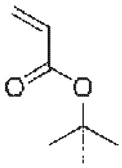 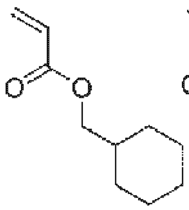 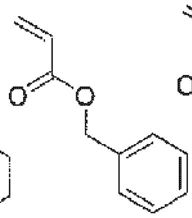 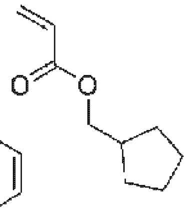 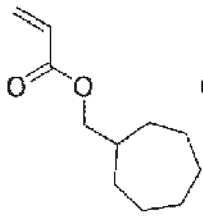 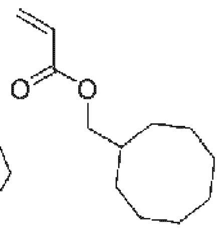

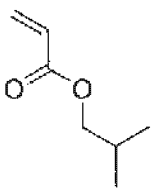 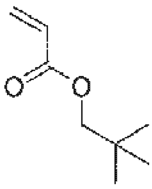 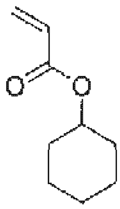 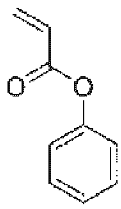 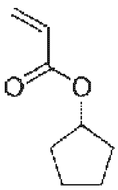 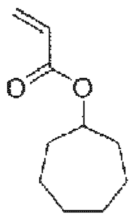 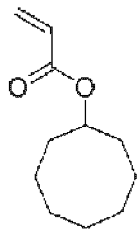

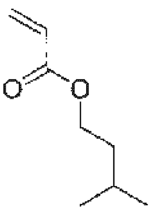 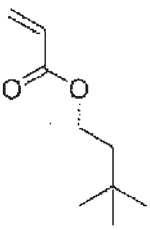 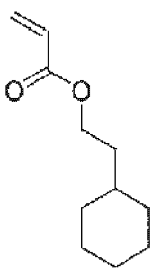 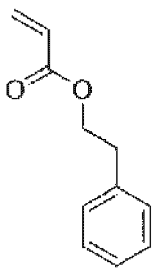 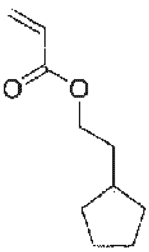 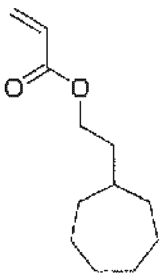 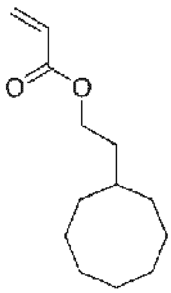

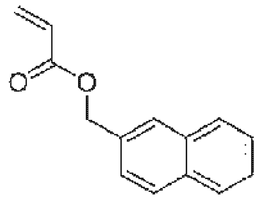
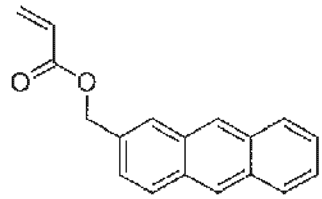
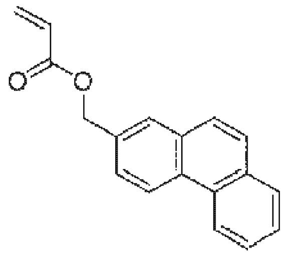
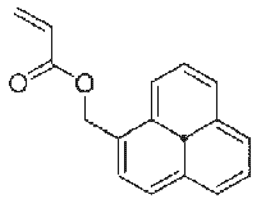

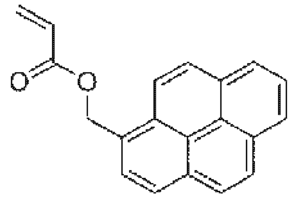
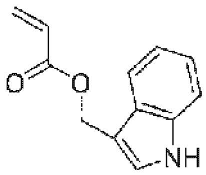
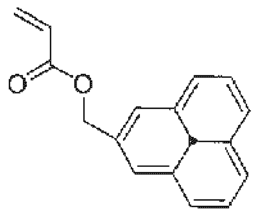
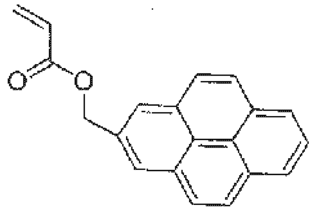

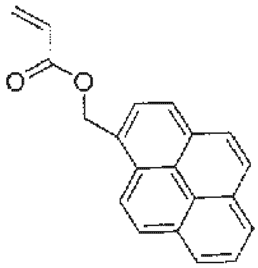
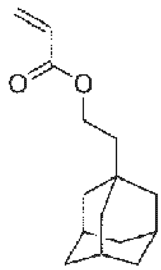

No text.

In general formula (1), $R^1$ represents a hydrogen atom or a $C_{1-3}$ alkyl group, and is preferably a hydrogen atom or a methyl group, preferably a hydrogen atom, an ethyl group, or a propyl group, and more preferably a hydrogen atom.

In general formula (2), $R^2$ represents a hydrogen atom or a $C_{1-3}$ alkyl group, and is preferably a hydrogen atom or a methyl group, preferably a hydrogen atom, an ethyl group, or a propyl group, and more preferably a hydrogen atom.

In general formula (3), $R^3$ represents a hydrogen atom or a $C_{1-3}$ alkyl group, and is preferably a hydrogen atom or a methyl group, preferably a hydrogen atom, an ethyl group, or a propyl group, and more preferably a hydrogen atom.

In general formula (1), $R^4$ represents a $C_{1-3}$ alkyl group, specifically a methyl group, an ethyl group, an n-propyl group or an isopropyl group, preferably a methyl group or an ethyl group, and more preferably a methyl group.

In general formula (1), $X^1$ represents an oxygen atom, a sulfur atom, or N-$R^7$, and is preferably an oxygen atom, a sulfur atom, or NH, and more preferably an oxygen atom.

In general formula (1), m represents an integer of 1 to 100, and is preferably an integer of 3 to 100, and preferably 3 to 80, more preferably 4 to 60, still more preferably 4 to 40, and even more preferably 4 to 22 from the viewpoint of imparting good hydrophilicity.

In general formula (2), $R^5$ represents a hydrogen atom, a $C_{1-18}$ alkyl group, a 3- to 8-membered cycloalkyl group optionally having a substituent, an adamantyl group, a $C_{6-18}$ aryl group optionally having a substituent, or a 5-to 10-membered heteroaryl group optionally having a substituent, and from the viewpoint of imparting hydrophobicity to the structural unit (B), a $C_{1-18}$ alkyl group, a 3- to 8-membered cycloalkyl group optionally having a substituent, an adamantyl group, a $C_{6-18}$ aryl group optionally having a substituent, or a 5- to 10-membered heteroaryl group optionally having a substituent is preferable, a $C_{1-18}$ alkyl group, a 3- to 8-membered cycloalkyl group optionally having a substituent, an adamantyl group, a $C_{6-18}$ aryl group optionally having a substituent, or a 5- to 10-membered heteroaryl group optionally having a substituent is more preferable, and a $C_{1-18}$ alkyl group, a 3- to 8-membered cycloalkyl group, an adamantyl group, or a $C_{6-18}$ aryl group is still more preferable. In addition, a 3- to 8-membered cycloalkyl group optionally having a substituent, an adamantyl group, a $C_{6-14}$ aryl group optionally having a substituent, or a 6-to 10-membered heteroaryl group optionally having a substituent is also preferable. Here, the substituent is preferably one or more selected from a halogen atom, an alkyl group having 1 to 6 carbon atoms, an alkenyl group having 2 to 6 carbon atoms, and an alkynyl group having 2 to 6 carbon atoms.

In general formula (2), $X^2$ represents an oxygen atom, a sulfur atom, or N-$R^7$, and is preferably an oxygen atom, a sulfur atom, or NH, and more preferably an oxygen atom.

In general formula (2), n represents an integer of 0 to 3, preferably an integer of 1 to 3, and more preferably 1.

In general formula (3), $R^6$ represents a hydrogen atom, a leaving group, or a linker. As the leaving group or linker, a $C_{1-18}$ alkyl group optionally having a substituent, a 3- to 8-membered cycloalkyl group optionally having a substituent, or a $C_{7-19}$ aralkyl group optionally having a substituent is preferable. Here, examples of the substituent include a halogen atom, an alkyl group having 1 to 6 carbon atoms, an alkenyl group having 2 to 6 carbon atoms, an alkynyl group having 2 to 6 carbon atoms, a hydroxyl group, an alkoxy group having 1 to 6 carbon atoms, an amino group, an alkylamino group having 1 to 6 carbon atoms, a di-alkylamino group having 1 to 6 carbon atoms in which alkyl groups are the same or different, a thiol group, an alkylthio group having 1 to 6 carbon atoms, a carboxyl group, an alkoxycarbonyl group having 1 to 6 carbon atoms, and a carbamoyl group. Among these groups, as the linker, a group having a functional group such as a hydroxyl group, an amino group, a thiol group, or a carboxyl group as a substituent is preferable.

Preferred specific examples of the leaving group of $R^6$ include a group represented by the following formula (4):

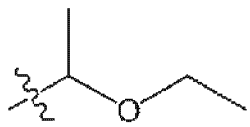

(4)

Preferred specific examples of the linker of $R^6$ include a group represented by the following formula (5):

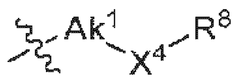

(5)

wherein, $R^8$ represents a hydrogen atom or the drug, $Ak^1$ represents a $C_{1-7}$ alkylene bond, and $X^4$ represents an oxygen atom, the sulfur atom, or $-N(R^7)-$ ($R^7$ represents a hydrogen atom or a $C_{1-3}$ alkyl group). A group represented by formula (5) in which $X^4$ represents an oxygen atom, a sulfur atom, or NH is more preferable.

More preferred examples of the linker of $R^6$ include groups selected from the following formulas (6) to (8).

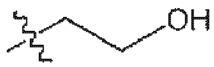

(6)

,

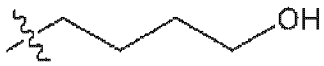

(7)

,

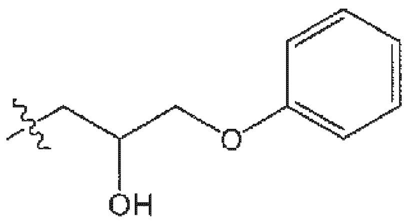

(8)

In general formula (3), $X^3$ represents an oxygen atom, a sulfur atom, or $N-R^7$, and is preferably an oxygen atom, a sulfur atom, or NH, and more preferably an oxygen atom or NH.

The copolymer X of the present invention is formed by copolymerizing three monomers represented by general formulas (1) to (3). The copolymerization may be random copolymerization or block copolymerization, and those formed by random copolymerization are preferable. As for a blending ratio of the three monomers, it is preferable that 0.01 to 100 parts by mass of monomer (2) and 0.1 to 100 parts by mass of monomer (3) are polymerized, it is more preferable that 0.05 to 18 parts by mass of monomer (2) and 0.1 to 20 parts by mass of monomer (3) are polymerized, and it is particularly preferable that 0.05 to 4 parts by mass of monomer (2) and 0.1 to 16 parts by mass of monomer (3) are polymerized, with respect to 1 part by mass of monomer (1).

In addition, "solvates" in which various solvents are coordinated are also included in the copolymer X of the present invention. In the present description, examples of the "solvate" include a hydrate and an ethanolate. The solvent may be coordinated to the copolymer X of the present invention in any number.

The copolymer X of the present invention can be produced by various known methods. The production method is not particularly limited, and for example, the copolymer X can be produced according to a basic polymer synthesis method described below.

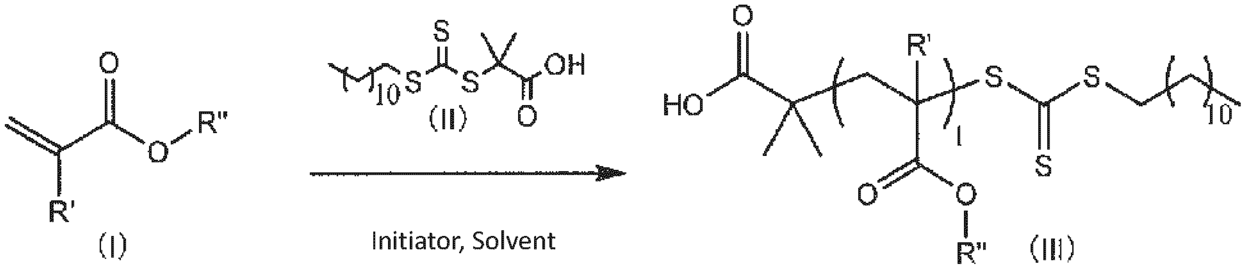


wherein R' represents a hydrogen atom or a $C_{1-3}$ alkyl group, and R" represents a group represented by $R^4$, $R^5$ or $R^6$.

This reaction shows a step of producing a polymer (III) by reacting a monomer (I) with a chain transfer agent (II) and an initiator. This reaction can be performed in the absence of a solvent or in a solvent such as alcohols such as methanol, ethanol, 1-propanol, and 2-propanol; ethers such as diethyl ether, tetrahydrofuran, and 1,4-dioxane; aromatic hydrocarbons such as benzene, toluene, and xylene; halogenated hydrocarbons such as dichloromethane, chloroform, and 1,2-dichloroethane; N,N-dimethylformamide; N,N-dimethylacetamide; N-methylpyrrolidone; acetonitrile; and ethyl acetate, and it is preferable to use aromatic hydrocarbons such as toluene and xylene as a solvent. As the chain transfer agent, for example, it is possible to use 2-(dodecylthiocarbonothioylthio)-2-methylpropionic acid (DDMAT), cyanomethyl dodecyltrithiocarbonate (CDTTC), 2-cyano-2-propyldodecyl trithiocarbonate (CPDTTC), 4-cyano-4-[(dodecylsulfanylthiocarbonyl)sulfanyl]pentanoic acid (CDSPA), or 2-(dodecylthiocarbonothioylthio)-2-methylpropionic acid 3-azido-1-propanol ester ($N_3$-CTA), and it is preferable to use DDMAT or $N_3$-CTA, and more preferably $N_3$-CTA. Where polymerization is carried out by using a chain transfer agent, the copolymer X of the present invention has a structure in which a part or all of a structure of the chain transfer agent is partially bonded. Where the copolymer X includes a structure of the chain transfer agent, the structure may be removed by an appropriate method. As the initiator, an azo polymerization initiator such as 2,2'-azobisisobutyronitrile (AIBN), 1,1'-azobis(cyclohexanecarbonitrile) (ACHN), 2,2'-azobis-2-methylbutyronitrile (AMBN), 2,2'-azobis-2,4-dimethylvaleronitrile (ADVN), or dimethyl 2,2'-azobis(2-methylpropionate) (MAIB) can be used, and AIBN is preferably used. The reaction temperature is 0 to 300°C, preferably 0 to 150°C, and more preferably 1 to 100°C, and the reaction time is 1 minute to 48 hours, and preferably 5 minutes to 24 hours. In this reaction, a random copolymerized copolymer X can be produced by carrying out the reaction in the coexistence of monomers (I) having different structures.

For example, if the $N_3$-CTA having an azide group is used as the chain transfer agent, the copolymer X having an end at which the azide group is present will be obtained, which is advantageous for production of the target-specific copolymer or the drug complex by a click reaction described later.

Examples of the target-specific molecule that binds to the copolymer X can include affibody molecules such as the anti-ErbB2 affibody (also known as anti-HER2 affibody), the anti-HER3 affibody, the anti-EGFR affibody, the anti-PD-L1 affibody, the anti-TNFα affibody, the anti-fibrinogen affibody, the anti-transferrin affibody, the anti-HSA affibody, the anti-insulin affibody, the anti-IgG affibody, the anti-IgM affibody, the anti-IgA affibody, and the anti-IgE affibody (for example, available from Abcam, Cambridge, MA.), and the anti-EGFR affibody, the anti-HER2 affibody, the anti-HER3 affibody, and the anti-PD-L1 affibody are more preferable.

Examples of preferred affibody molecules can include the molecules described in WO 2009/080810 A (anti-HER2), WO 2011/056124 A (anti-HER3), WO 2014/053586 A (anti-HER3), WO 2007/065635 A (anti-EGFR), and WO 2017/072280 A (anti-PD-L1). The affibody molecule may be modified with one or more amino acids to the extent that the binding specificity is maintained.

A more preferred affibody molecule, as the anti-HER2 affibody, includes a sequence represented by the following amino acid sequence (2) known as Z(HER2:2891).
AEAKYAKEMRNAYWEIALLPNLTNQQKRAFIRKLYDDPSQSSELLSEAKKLNDSQAPK (2)

The anti-HER3 affibody includes a sequence represented by the following amino acid sequence (3) known as Z(HER3: Z08699).
AEAKYAKEKYNAYYEIWQLPNLTKYQKAAFIGKLQDDPSQSSELLSEAKKLNDSQAPK (3)

The anti-EGFR affibody include a sequence represented by the following amino acid sequence (4) known as Z(EGFR: 1907),

VDNKFNKEMWAAWEEIRNLPNLNGWQMTAFIASLVDDPSQSANLLAEAKKLNDAQAPK (4)
a sequence represented by the following amino acid sequence (5) known as Z(EGFR:2377),
VDNKFNKEMWIAWEEIRDLPNLNGWQMTAFIASLLDDPSQSANLLAEAKKLNDAQAPK (5)
a sequence represented by the following amino acid sequence (6) as Z(EGFR:03115),
AEAKYAKEMWIAWEEIRNLPNLNGWQMTAFIAKLLDDPSQSSELLSEAKKLNDSQAPK (6)
an affibody molecule used in ABY-029, a sequence represented by the following amino acid sequence (7) as Z(PD-L1:18609),
AEAKYAKERNAAAYEILYLPNLTNAQKWAFIWKLDDDPSQSSELLSEAKKLNDSQAPK (7)
or a sequence represented by the following amino acid sequence (8) as Z(PD-L1:18608).
AEAKYAKERNKAAYEILYLPNLTNAQKWAFIWKLDDDPSQSSELLSEAKKLNDSQAPK (8)

The affibody molecule may be modified with one or more amino acids as long as the binding specificity is maintained. Examples thereof include a 6 × His tag, a 3 × HE tag, and VDC, and the affibodies including these sequences can be isolated and purified by a similar procedure as a method for recovering a tagged protein.
Further, these affibody molecules can be bonded to the copolymer X via a Cys residue or an amino group of an amino acid in the sequences, respectively.
Where geometric isomers or optical isomers are present in the compounds of the present invention, mixtures or separations of those isomers are also included in the scope of the present invention. Separation of the isomers can be performed by a conventional method.
The target-specific copolymer or the drug complex of the present invention can be produced by various known methods. The production method is not particularly limited, and for example, the target-specific copolymer or the drug complex can be produced according to a synthesis method through the click reaction described below.

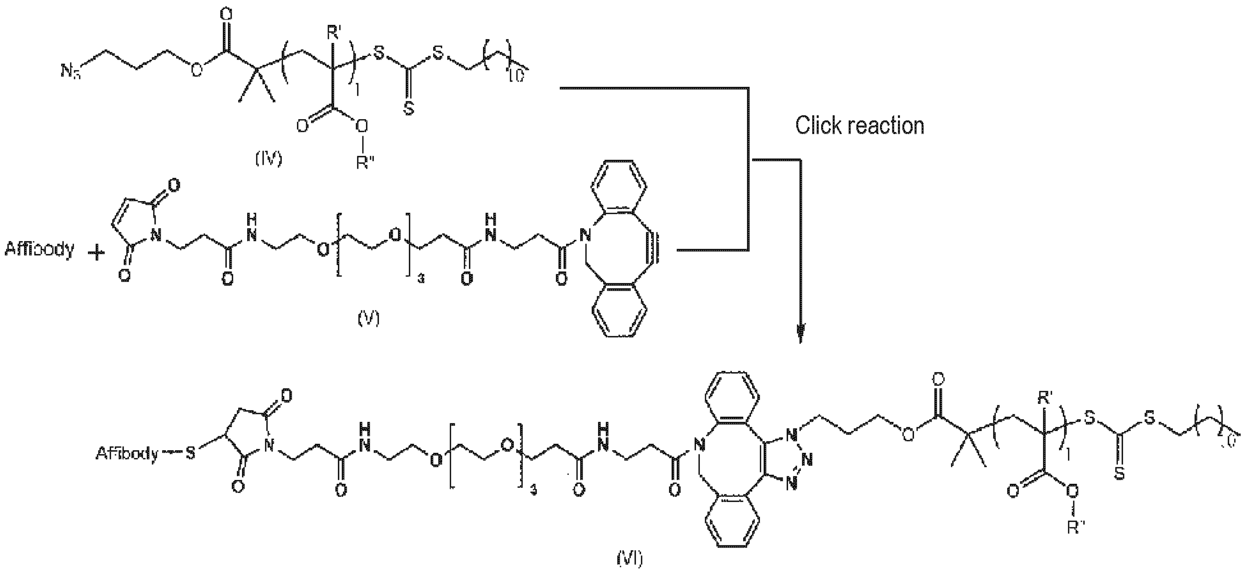


wherein R' represents a hydrogen atom or a $C_{1-3}$ alkyl group, and R" represents a group represented by $R^4$, $R^5$ or $R^6$.
For complexing the target-specific molecule with SCNP by the covalent bond, a functional group for a complexation reaction is introduced onto a surface of SCNP, and the complexation can be performed through a reaction with the SH group, the amino group, or the carboxyl group at ends or side chains of the target-specific molecules that can react with the functional group. It is also possible to introduce a spacer into the ends or the side chains of the target-specific molecule with an appropriate crosslinking reagent (crosslinker) to form a covalent bond to SCNP as a modified target-specific molecule. Examples of such bond formation include click chemistry between an azide group and an alkyne, a reaction between a sulfhydryl group and a maleimide group, and a reaction between an amino group and a succinimidyl group.
The "click chemistry" is classified to reactions similar to natural biochemical reactions, having the following attributes, and has the following characteristics. The click chemistry is a very efficient reaction that proceeds rapidly to obtain high yields, and is a very selective reaction that produces no (or little) by-products and allows polyfunctional groups. Moreover, the click chemistry proceeds under mild reaction conditions such as a low temperature (or ambient temperature) or in an aqueous solution. This reaction can be performed in water or in a solvent such as an alcohol such as methanol, ethanol, 1-propanol, or 2-propanol; an ether such as diethyl ether, tetrahydrofuran, or 1,4-dioxane; an aromatic hydrocarbon such as benzene, toluene, or xylene; a halogenated hydrocarbon such as dichloromethane, chloroform, or 1,2-dichloroethane; N,N-dimethylformamide; N,N-dimethylacetamide; N-methylpyrrolidone; acetonitrile; or ethyl acetate. It is preferable to use water or N,N-dimethylformamide as the solvent. In some aspects, the cyclooctyne is dibenzocyclooctyne (DBCO), difluorobenzocyclooctyne (DIFBO), biarylazacyclooctynone (BARAC), dibenzocyclooctyne (DIBO), difluorinated cyclooctyne (DIFO), monofluorinated cyclooctyne (MOFO), dimethoxyazacyclooctyne (DIMAC), or aryl-less octyne (ALO), with dibenzocyclooctyne (DBCO) being preferably used. In some aspects, the alkyne is aliphatic alkyne and the reacting step is performed in the presence of a copper (I) catalyst. In some aspects, the alkyne is cyclooctyne and the

reacting step is performed under copper-free conditions. The reaction temperature is 0 to 300°C, preferably 0 to 150°C, and more preferably 1 to 100°C, and the reaction time is 1 minute to 48 hours, and preferably 5 minutes to 24 hours.

Reaction of the reaction formula is Huisgen cycloaddition which is a kind of click reaction, and is a 1,3-dipolar cycloaddition reaction in which 1,2,3-triazole is formed from the azide and the alkyne.

The produced polymer X, target-specific copolymer, and drug complex of the present invention can be purified by a polymer isolation and purification method generally known in the field of polymer chemistry. Specific examples thereof include treatment operations such as extraction, recrystallization, salting out using, for example, ammonium sulfate or sodium sulfate, centrifugation, dialysis, ultrafiltration, adsorption chromatography, ion exchange chromatography, hydrophobic chromatography, normal phase chromatography, reverse phase chromatography, desalting column chromatography, gel filtration, gel permeation chromatography, affinity chromatography, electrophoresis, countercurrent distribution, and combinations thereof. In particular, in the hydrophobic chromatography, since retention time changes according to the number of SCNP bonded to one target-specific molecule, it is possible to collect a fraction satisfying any DAR by fractionation.

The copolymer X and the target-specific copolymer of the present invention can be utilized as carriers for transporting various physiologically active substances (drugs). For example, a pharmaceutical composition in which a tumor therapeutic agent is carried on (encapsulated in) the copolymer X, the target-specific copolymer, or the drug complex of the present invention can be used as a prophylactic and/or therapeutic agent for various cancer diseases such as a colon cancer, a duodenal cancer, a gastric cancer, a pancreatic cancer, a liver cancer, a lung cancer, a uterine cancer, and an ovarian cancer, because the pharmaceutical composition suppresses growth of tumors, as confirmed in test examples described later. Further, with a high tumor accumulation ability, the pharmaceutical composition can be used as a tumor diagnostic agent or a contrast agent.

When the copolymer and the target-specific copolymer of the present invention are used as a drug transport carrier, the dose and the number of doses may be appropriately selected in consideration of, for example, administration form, age and body weight of a patient, and nature or severity of a symptom to be treated, and the dose and the number of doses should not be limited. However, when a polymer encapsulating a drug or the drug complex is intravenously injected by an injection, for example, for an adult (60 kg), a single dose is preferably administered in an amount of 0.12 mg to 12 000 000 mg, more preferably 1.2 mg to 1 200 000 mg, and particularly preferably 12 to 120 000 mg.

The pharmaceutical composition of the present invention can be produced by mixing the drug with the copolymer X, the target-specific copolymer, or the drug complex of the present invention. Preferably, the copolymer, the target-specific copolymer, or the drug complex of the present invention may be mixed with the drug to produce the single chain nanoparticle, or the single chain nanoparticle of the copolymer X, the target- specific copolymer, or the drug complex of the present invention may be produced and then mixed with the drug. The single chain nanoparticle can be produced by a known method.

In the pharmaceutical composition of the present invention, the drug may be carried on the copolymer X, the target-specific copolymer, or the drug complex by the action such as electrostatic interaction, hydrogen bond, hydrophobic interaction, or covalent bond.

The drug is preferably an anticancer agent, more preferably an anticancer agent that acts on cancer cells to suppress proliferation of the cancer cells, and examples thereof include an antimetabolite, an alkylating agent, an anthracycline, an antibiotic, a mitotic inhibitor, a topoisomerase inhibitor, a proteasome inhibitor, and an anti-hormone agent.

Examples of the antimetabolite include azathioprine, 6-mercaptopurine, 6-thioguanine, fludarabine, pentostatin, cladribine, 5-fluorouracil (5FU), floxuridine (FUDR), cytosine arabinoside (cytarabine), methotrexate, trimethoprim, pyrimethamine, and pemetrexed. Examples of the alkylating agent include cyclophosphamide, mechlorethamine, uramustine, melphalan, chlorambucil, thiotepa/chlorambucil, ifosfamide, carmustine, lomustine, streptozocin, busulfan, dibromomannitol, cisplatin, carboplatin, nedaplatin, oxaliplatin, miriplatin, satraplatin, triplatin tetranitrate, procarbazine, altretamine, dacarbazine, mitozolomide, trabectedin, and temozolomide. Examples of the anthracycline include daunorubicin, doxorubicin, epirubicin, idarubicin, valrubicin, aclarubicin, amrubicin, and pirarubicin. Examples of the antibiotic include dactinomycin, bleomycin, mithramycin, anthramycin, streptozotocin, gramicidin D, staurosporine, mitomycins (for example, mitomycin C), duocarmycins (for example, CC-1065), and calicheamicins. Examples of the mitotic inhibitor include maytansinoids (for example, DM0, mertansine (also known as DM1), DM2, DM3, DM4, or emtansine), auristatins (for example, auristatin E, auristatin phenylalanine phenylenediamine (AFP), monomethyl auristatin E, monomethyl auristatin D, or monomethyl auristatin F), dolastatins, cryptophycins, vinca alkaloid (for example, vincristine, vinblastine, vindesine, or vinorelbine), taxanes (for example, paclitaxel or docetaxel), and colchicines. Examples of the topoisomerase inhibitor include irinotecan, topotecan, nogitecan, amsacrine, etoposide, teniposide, mizantrone, mitoxantrone, SN-38, exatecan, and deruxtecan. Examples of the proteasome inhibitor can include a peptidyl boronic acid, carfilzomib, and bortezomib. Examples of the anti-hormone agent can include fulvestrant, tamoxifen, and toremifene. Where these drugs are prepared into the pharmaceutical composition of the present invention, one or more of the drugs can also be used in combination, and the drugs may be carried on the copolymer as a free form.

A route of administration of the pharmaceutical composition of the present invention is desirably the most effective one for treatment, and the pharmaceutical composition can be administered by a parenteral administration preparation such as an oral administration preparation, an injection, or a transdermal administration preparation. For example, parenteral administration such as intraarterial injection, intravenous injection, subcutaneous injection, intramuscular injection, or intraperitoneal injection is preferable, and intraarterial injection and intravenous injection are more preferable. The number of doses should not be limited, and examples thereof include one to several doses per week average.

Various preparations suitable for the route of administration can be produced by a conventional method by appropriately selecting preparation additives such as excipients, extenders, binders, wetting agents, disintegrants, lubricants, surfactants, dispersants, buffers, preservatives, solubilizing agents, antiseptics, flavoring agents, soothing agents, stabilizers, and isotonizing agents that are conventionally used in formulation.

The preparation additives that can be contained in the various preparations described above are not particularly limited as long as the preparation additives are pharmaceutically acceptable. Examples of such preparation additives include purified water, water for injection, distilled water for injection, pharmaceutically acceptable organic solvents, collagen, polyvinyl alcohol, polyvinylpyrrolidone, carboxyvinyl polymer, sodium alginate, water-soluble dextran, sodium carboxymethyl starch, pectin, xanthan gum, gum arabic, casein, gelatin, agar, glycerin, propylene glycol, polyethylene glycol, petrolatum, paraffin, stearyl alcohol, stearic acid, human serum albumin, mannitol, sorbitol, and lactose. Additives to be used are appropriately selected according to various preparations and can be used alone or in combination.

The injection can also be prepared as a non-aqueous diluent (for example, polyethylene glycol, vegetable oils such as olive oil, and alcohols such as ethanol), a suspension, or an emulsion. Sterilization of the injection can be performed by filtration sterilization using a filter, and blending of, for example, a microbicide. In addition, the injection can be produced in a form of preparation before use. That is, the injection can be formed into a sterile solid composition by, for example, lyophilization, and can be dissolved in water for injection, distilled water for injection, or another solvent before use.

## Examples

Hereinafter, the present invention will be described more specifically with reference to examples. These examples are provided for purpose of exemplification and are not intended to limit embodiments of the invention.

### [Example 1] Production of poly[(benzyl acrylate)-co-(poly(ethylene glycol)methyl ether acrylate)-co-(1-ethoxyethyl acrylate)]

(1) Synthesis of 1-ethoxyethyl acrylate (EEA)

Ethyl vinyl ether (28.725 mL) was weighed under an argon atmosphere, and thereto was added a phosphoric acid (50 mg) under ice cooling. Thereto was added acrylic acid (17.15 mL), and the mixture was stirred at room temperature for 48 hours. Further thereto was added hydrotalcite (3 g), and the mixture was stirred for 2 hours, and the reaction was stopped. After celite filtration, unreacted ethyl vinyl ether was removed by evaporation. Thereto was added phenothiazine (up to 500 ppm) as a polymerization inhibitor, and the mixture was purified by distillation under a reduced pressure together with calcium hydride (distillation temperature of 28 to 32°C). The obtained 1-ethoxyethyl acrylate was dispensed into a glass vial and stored at -30°C.

$^{13}C$ NMR (400MHz, $CDCl_3$), is, ppm: 15.29 ($-OCH_2CH_3$), 21.16 ($-COOCH(CH_3)$), 64.98 ($-OCH_2-$), 96.73 ($-COOCH(CH_3)$ 128.84 ($CH_2CH-$), 131.43 ($CH_2CH-$), 166.00 (-COO).

(2) Synthesis of poly[(benzyl acrylate)-co-(poly(ethylene glycol) methyl ether acrylate)-co-(1-ethoxyethyl acrylate)]

100 mg of 2-(dodecylthiocarbonothioylthio)-2-methylpropionic acid (DDMAT) was weighed and dissolved in 17.3 mL of toluene to prepare a DDMAT/toluene stock solution (5.78 mg/mL as a DDMAT concentration). Similarly, 22 mg of 2,2'-azobis(2-methylpropionitrile) (AIBN) was weighed and dissolved in 17.3 mL of toluene to prepare an AIBN/toluene stock solution (AIBN concentration: 1.27 mg/mL). Separately, 1.296 g of poly(ethylene glycol) methyl ether acrylate (mPEGA, the average value (n) of the numbers of repetitions of ethylene glycol is 9), 0.394 g of benzyl acrylate (BnA), 0.039 g of 1-ethoxyethyl acrylate, 1.73 mL of a DDMAT/toluene stock solution, and 1.73 mL of an AIBN/toluene stock solution were added, and polymerization was performed in an oil bath at 70°C. After a lapse of 90 minutes, the polymerization was stopped, and then the reaction solution was subjected to a reprecipitation method or dialyzed against methanol to recover the copolymer. Since the obtained copolymer was basically a viscous body, in the reprecipitation method, an operation of dropping the reaction solution into a centrifuge tube to which a poor solvent (hexane/ethyl acetate = 7/3 [v/v]) was added and recovering the solution by centrifugation (2 000 × g, 5 min) was repeated 3 times, and finally

vacuum drying was performed to obtain 1.223 g of poly[(benzyl acrylate)-co-(poly(ethylene glycol) methyl ether acrylate)-co-(1-ethoxyethyl acrylate)].

As a result of analyzing the polymerization degree of each monomer and the number average molecular weight ($M_{n,NMR}$) from a $^1$H-NMR spectrum of the obtained copolymer measured using NMR, the polymerization degree of mPEGA (n = 9) was 102, the polymerization degree of BnA was 94, the polymerization degree of EEA was 9, and $M_{n,NMR}$ was 65 900. The molecular weight dispersion ($M_w/M_n$) of the obtained copolymer was measured using GPC, and as a result, it was found to be 1.53.

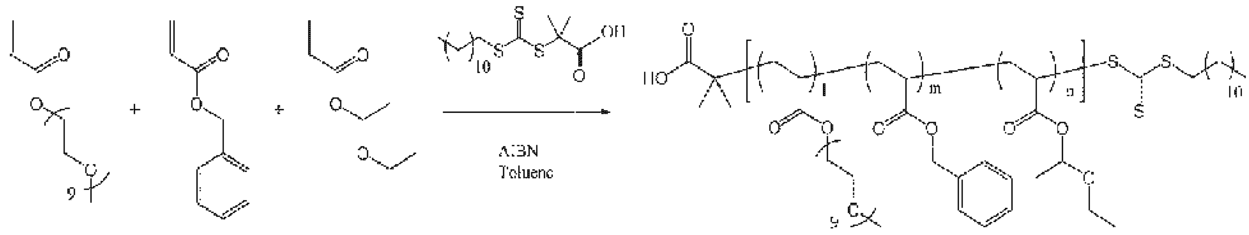


[Measurement apparatuses and conditions]

(1) $^1$H-NMR measurement

Apparatus: JNM-ECX400 (400 MHz)/JEOL Ltd.
Solvent: dimethyl sulfoxide-$d_6$ containing 0.03% tetramethylsilane/KANTO CHEMICAL CO., INC.
Sample concentration: 20 mg/mL
Measurement temperature: 25°C
Number of integration times: 256 times
Results: Fig. 1

(2) GPC measurement

Apparatus: HPLC-Prominence system/SHIMADZU CORPORATION
Detector: RID-10A Refractive index detector/SHIMADZU CORPORATION
Column: TSKgel α-2500 column/Tosoh Corporation
(Column size: 7.8 mm × 300 mm, particle size: 7 um, and exclusion limit molecular weight: 5 × $10^3$)
TSKgel α-4000 column/Tosoh Corporation
(Column size: 7.8 mm × 300 mm, particle size: 10 μm, and exclusion limit molecular weight: 4 × $10^5$)
TSKgel guardcolum/Tosoh Corporation
Mobile phase: N,N-dimethyformamide (DMF) containing 10 mmol/L of lithium bromide
Temperature: 40°C
Flow rate: 0.5 mL/min
Sample concentration: 6 mg/mL
Standard substance: poly(methyl methacrylate) standard ReadyCal set, $M_p$ 800 - 2 200 000 Da/SIGMA
Results: Fig. 2

[Table 1]

| Example | Composition ratio (molar ratio to chain transfer agent) | | | | | Solvent | Temperature (°C) | Polymerization time (min) | Polymerization degree | | | $M_{n,NMR}$ | $M_w/M_n$ |
|---|---|---|---|---|---|---|---|---|---|---|---|---|---|
| | Chain transfer agent | Initiator | Monomer | | | | | | | | | | |
| | DDMAT | AIBN | mPEGA n=9 | BnA | EEA | | | | mPEGA n=9 | BnA | EEA | | |
| 1 | 1 | 0.5 | 100 | 90 | 10 | Toluene | 70 | 90 | 102 | 94 | 9 | 65 900 | 1.53 |

[Examples 2 to 68]

Polymers having different composition ratios and average molecular weights as shown in the following table were produced by appropriately changing types, charged amounts, reaction temperatures, and polymerization times of the monomers (mPEGA, BnA, and EEA) used in Example 1, and using the same method as in Example 1.

[Table 2]

| Example | Composition ratio (molar ratio to chain transfer agent) | | | | | | | | | | | Solvent | Temperature (°C) | Polymerization time (min) |
|---|---|---|---|---|---|---|---|---|---|---|---|---|---|---|
| | Chain transfer agent | Initiator | | Monomer | | | | | | | | | | |
| | DDMAT | AIBN | ACHN | mPEGA n=4 | mPEGA n=9 | mPEGA n=22 | BnA | EEA | 2-Hydroxy ethyl acrylate | 4-Hydroxy butyl acrylate | 2-Hydroxy-3-phenoxy propyl acrylate | | | |
| 2 | 1 | 2 | - | 255 | - | - | 15 | 30 | - | - | - | Toluene | 70 | 90 |
| 3 | 1 | 2 | - | 160 | - | - | 20 | 20 | - | - | - | Toluene | 70 | 60 |
| 4 | 1 | 2 | - | 240 | - | - | 30 | 30 | - | - | - | Toluene | 70 | 60 |
| 5 | 1 | 2 | - | 170 | - | - | 10 | 20 | - | - | - | Toluene | 70 | 60 |
| 6 | 1 | 2 | - | 255 | - | - | 15 | 30 | - | - | - | Toluene | 70 | 60 |
| 7 | 1 | 0.5 | - | - | 16 | - | 16 | 8 | - | - | - | Toluene | 70 | 90 |
| 8 | 1 | 0.5 | - | - | 18 | - | 14 | 8 | - | - | - | Toluene | 70 | 90 |
| 9 | 1 | 0.5 | - | - | 20 | - | 12 | 8 | - | - | - | Toluene | 70 | 90 |
| 10 | 1 | 0.5 | - | - | 20 | - | 12 | 8 | - | - | - | Toluene | 70 | 90 |
| 11 | 1 | 0.5 | - | - | 20 | - | 12 | 8 | - | - | - | Toluene | 70 | 90 |
| 12 | 1 | 0.5 | - | - | 20 | - | 16 | 4 | - | - | - | Toluene | 70 | 10 |
| 13 | 1 | 0.5 | - | - | 20 | - | 16 | 4 | - | - | - | Toluene | 70 | 30 |
| 14 | 1 | 0.5 | - | - | 20 | - | 16 | 4 | - | - | - | Toluene | 70 | 50 |
| 15 | 1 | 0.5 | - | - | 20 | - | 16 | 4 | - | - | - | Toluene | 70 | 70 |
| 16 | 1 | 0.5 | - | - | 20 | - | 16 | 4 | - | - | - | Toluene | 70 | 90 |

| | | | | | | | | | | | | | | |
|---|---|---|---|---|---|---|---|---|---|---|---|---|---|---|
| 17 | 1 | 0.5 | - | - | 22 | - | 10 | 8 | - | - | - | Toluene | 70 | 90 |
| 18 | 1 | 0.5 | - | - | 24 | - | 8 | 8 | - | - | - | Toluene | 70 | 90 |
| 19 | 1 | 0.5 | - | - | 24 | - | 8 | 8 | - | - | - | Toluene | 70 | 90 |
| 20 | 1 | 0.5 | - | - | 28 | - | 4 | 8 | - | - | - | Toluene | 70 | 90 |
| 21 | 1 | 0.5 | - | - | 10 | - | 25 | 15 | - | - | - | Toluene | 70 | 90 |
| 22 | 1 | 0.5 | - | - | 17 | - | 25 | 8 | - | - | - | Toluene | 70 | 90 |
| 23 | 1 | 0.5 | - | - | 22.5 | - | 12.5 | 15 | - | - | - | Toluene | 70 | 90 |
| 24 | 1 | 0.5 | - | - | 25 | - | 20 | 5 | - | - | - | Toluene | 70 | 90 |
| 25 | 1 | 0.5 | - | - | 29.5 | - | 12.5 | 8 | - | - | - | Toluene | 70 | 90 |
| 26 | 1 | 0.5 | - | - | 30 | - | 24 | 6 | - | - | - | Toluene | 70 | 90 |
| 27 | 1 | 0.5 | - | - | 35 | - | 28 | 7 | - | - | - | Toluene | 70 | 90 |
| 28 | 1 | 0.5 | - | - | 50 | - | 40 | 10 | - | - | - | Toluene | 70 | 90 |
| 29 | 1 | 0.5 | - | - | 10 | - | 10 | 180 | - | - | - | Toluene | 70 | 90 |
| 30 | 1 | 0.5 | - | - | 30 | - | 30 | 140 | - | - | - | Toluene | 70 | 90 |
| 31 | 1 | 0.5 | - | - | 30 | - | 70 | 100 | - | - | - | Toluene | 70 | 90 |
| 32 | 1 | 0.5 | - | - | 30 | - | 110 | 60 | - | - | - | Toluene | 70 | 90 |
| 33 | 1 | 0.5 | - | - | 50 | - | 10 | 140 | - | - | - | Toluene | 70 | 90 |
| 34 | 1 | 0.5 | - | - | 50 | - | 50 | 100 | - | - | - | Toluene | 70 | 90 |
| 35 | 1 | 0.5 | - | - | 50 | - | 90 | 60 | - | - | - | Toluene | 70 | 90 |
| 36 | 1 | 0.5 | - | - | 50 | - | 130 | 20 | - | - | - | Toluene | 70 | 90 |
| 37 | 1 | 0.5 | - | - | 70 | - | 30 | 100 | - | - | - | Toluene | 70 | 90 |
| 38 | 1 | 0.5 | - | - | 70 | - | 70 | 60 | - | - | - | Toluene | 70 | 90 |
| 39 | 1 | 0.5 | - | - | 70 | - | 110 | 20 | - | - | - | Toluene | 70 | 90 |
| 40 | 1 | 0.5 | - | - | 80 | - | 80 | 40 | - | - | - | Toluene | 70 | 90 |
| 41 | 1 | 0.5 | - | - | 80 | - | 100 | 20 | - | - | - | Toluene | 70 | 90 |
| 42 | 1 | 0.5 | - | - | 90 | - | 10 | 100 | - | - | - | Toluene | 70 | 90 |
| 43 | 1 | 0.5 | - | - | 90 | - | 50 | 60 | - | - | - | Toluene | 70 | 90 |
| 44 | 1 | 0.5 | - | - | 90 | - | 90 | 20 | - | - | - | Toluene | 70 | 90 |
| 45 | 1 | 0.5 | - | - | 100 | - | 20 | 80 | - | - | - | Toluene | 70 | 90 |
| 46 | 1 | 0.5 | - | - | 100 | - | 40 | 60 | - | - | - | Toluene | 70 | 90 |
| 47 | 1 | 0.5 | - | - | 100 | - | 50 | 50 | - | - | - | Toluene | 70 | 90 |
| 48 | 1 | 0.5 | - | - | 100 | - | 60 | 40 | - | - | - | Toluene | 70 | 90 |
| 49 | 1 | 0.5 | - | - | 100 | - | 70 | 30 | - | - | - | Toluene | 70 | 90 |
| 50 | 1 | 0.5 | - | - | 100 | - | 80 | 20 | - | - | - | Toluene | 70 | 90 |
| 51 | 1 | 0.5 | - | - | 110 | - | 30 | 60 | - | - | - | Toluene | 70 | 90 |
| 52 | 1 | 0.5 | - | - | 110 | - | 70 | 20 | - | - | - | Toluene | 70 | 90 |
| 53 | 1 | 0.5 | - | - | 130 | - | 10 | 60 | - | - | - | Toluene | 70 | 90 |
| 54 | 1 | 0.5 | - | - | 130 | - | 50 | 20 | - | - | - | Toluene | 70 | 90 |
| 55 | 1 | 0.5 | - | - | 150 | - | 30 | 20 | - | - | - | Toluene | 70 | 90 |
| 56 | 1 | 0.5 | - | - | 170 | - | 10 | 20 | - | - | - | Toluene | 70 | 90 |
| 57 | 1 | 0.5 | - | - | 200 | - | 160 | 40 | - | - | - | Toluene | 70 | 90 |
| 58 | 1 | 0.5 | - | - | 300 | - | 240 | 60 | - | - | - | Toluene | 70 | 90 |
| 59 | 1 | 0.5 | - | - | 400 | - | 320 | 80 | - | - | - | Toluene | 70 | 90 |
| 60 | 1 | 2 | - | - | - | 105 | 155 | 40 | - | - | - | Toluene | 70 | 90 |
| 61 | 1 | 2 | - | - | - | 120 | 240 | 40 | - | - | - | Toluene | 70 | 90 |
| 62 | 1 | 2 | - | - | - | 140 | 210 | 50 | - | - | - | Toluene | 70 | 90 |

| | | | | | | | | | | | | | | |
|---|---|---|---|---|---|---|---|---|---|---|---|---|---|---|
| 63 | 1 | 2 | - | - | - | 140 | 220 | 40 | - | - | - | Toluene | 70 | 90 |
| 64 | 1 | 0.1 | - | - | 100 | - | 80 | - | 20 | - | - | 1,4-dioxane | 70 | 90 |
| 65 | 1 | - | 0.5 | - | 100 | - | 50 | - | - | 50 | - | 1,4-dioxane | 70 | 180 |
| 66 | 1 | - | 0.5 | - | 100 | - | 80 | - | - | 20 | - | 1,4-dioxane | 70 | 180 |
| 67 | 1 | 0.1 | - | - | 130 | - | 65 | - | - | - | 65 | 1,4-dioxane | 70 | 90 |
| 68 | 1 | 0.1 | - | - | 130 | - | 104 | - | - | - | 26 | 1,4-dioxane | 70 | 90 |

[Table 3]

| Example | Polymerization degree | | | | | | | | $M_{n,NMR}$ | $M_w/M_n$ |
|---|---|---|---|---|---|---|---|---|---|---|
| | mPEGA | | | BnA | EEA | 2-Hydroxy ethyl acrylate | 4-Hydroxy butyl acrylate | 2-Hydroxy-3-phenoxy propyl acrylate | | |
| | n=4 | n=9 | n=22 | | | | | | | |
| 2 | 250 | - | - | 18 | 31 | - | - | - | 62 400 | 1.37 |
| 3 | 155 | - | - | 22 | 20 | - | - | - | 39 200 | 1.23 |
| 4 | 211 | - | - | 29 | 29 | - | - | - | 53 100 | 1.32 |
| 5 | 162 | - | - | 11 | 20 | - | - | - | 39 000 | 1.22 |
| 6 | 227 | - | - | 15 | 28 | - | - | - | 54 300 | 1.33 |
| 7 | - | 16 | - | 16 | 8 | - | - | - | 11 800 | 1.20 |
| 8 | - | 18 | - | 14 | 8 | - | - | - | 12 400 | 1.20 |
| 9 | - | 13 | - | 8 | 5 | - | - | - | 8 600 | 1.46 |
| 10 | - | 20 | - | 13 | 8 | - | - | - | 13 400 | 1.30 |
| 11 | - | 18 | - | 11 | 7 | - | - | - | 11 700 | 1.33 |
| 12 | - | 8 | - | 7 | 1 | - | - | - | 5 300 | 1.51 |
| 13 | - | 16 | - | 13 | 3 | - | - | - | 10 800 | 1.30 |
| 14 | - | 19 | - | 15 | 3 | - | - | - | 12 200 | 1.28 |
| 15 | - | 20 | - | 16 | 4 | - | - | - | 13 300 | 1.39 |
| 16 | - | 24 | - | 19 | 4 | - | - | - | 15 700 | 1.29 |
| 17 | - | 21 | - | 10 | 8 | - | - | - | 13 200 | 1.20 |
| 18 | - | 13 | - | 5 | 4 | - | - | - | 8 000 | 1.44 |
| 19 | - | 24 | - | 8 | 8 | - | - | - | 14 300 | 1.21 |
| 20 | - | 26 | - | 4 | 8 | - | - | - | 14 600 | 1.20 |
| 21 | - | 7 | - | 17 | 9 | - | - | - | 8 100 | 1.41 |
| 22 | - | 11 | - | 17 | 5 | - | - | - | 9 200 | 1.39 |
| 23 | - | 15 | - | 9 | 10 | - | - | - | 10 700 | 1.30 |
| 24 | - | 26 | - | 21 | 5 | - | - | - | 16 900 | 1.31 |
| 25 | - | 20 | - | 10 | 6 | - | - | - | 12 400 | 1.33 |
| 26 | - | 33 | - | 27 | 6 | - | - | - | 21 500 | 1.34 |
| 27 | - | 39 | - | 30 | 7 | - | - | - | 25 000 | 1.36 |
| 28 | - | 39 | - | 4 | 8 | - | - | - | 20 200 | 1.25 |
| 29 | - | 10 | - | 10 | 159 | - | - | - | 29 700 | 1.19 |
| 30 | - | 27 | - | 27 | 122 | - | - | - | 35 600 | 1.27 |
| 31 | - | 30 | - | 66 | 91 | - | - | - | 38 600 | 1.17 |

| | | | | | | | | | | |
|---|---|---|---|---|---|---|---|---|---|---|
| 32 | - | 27 | - | 91 | 52 | - | - | - | 35 800 | 1.20 |
| 33 | - | 45 | - | 10 | 123 | - | - | - | 41 000 | 1.31 |
| 34 | - | 44 | - | 45 | 86 | - | - | - | 41 100 | 1.20 |
| 35 | - | 45 | - | 79 | 54 | - | - | - | 42 700 | 1.24 |
| 36 | - | 43 | - | 111 | 19 | - | - | - | 42 000 | 1.28 |
| 37 | - | 60 | - | 28 | 87 | - | - | - | 46 500 | 1.24 |
| 38 | - | 55 | - | 57 | 49 | - | - | - | 43 000 | 1.27 |
| 39 | - | 56 | - | 89 | 18 | - | - | - | 44 300 | 1.30 |
| 40 | - | 79 | - | 80 | 36 | - | - | - | 56 400 | 1.43 |
| 41 | - | 81 | - | 106 | 17 | - | - | - | 58 900 | 1.51 |
| 42 | - | 81 | - | 12 | 93 | - | - | - | 54 600 | 1.27 |
| 43 | - | 71 | - | 42 | 51 | - | - | - | 48 600 | 1.30 |
| 44 | - | 70 | - | 73 | 18 | - | - | - | 48 400 | 1.32 |
| 45 | - | 84 | - | 19 | 72 | - | - | - | 54 200 | 1.33 |
| 46 | - | 80 | - | 35 | 52 | - | - | - | 51 900 | 1.35 |
| 47 | - | 84 | - | 45 | 44 | - | - | - | 54 200 | 1.33 |
| 48 | - | 82 | - | 52 | 35 | - | - | - | 53 100 | 1.36 |
| 49 | - | 94 | - | 68 | 26 | - | - | - | 60 100 | 1.50 |
| 50 | - | 104 | - | 83 | 20 | - | - | - | 66 700 | 1.52 |
| 51 | - | 93 | - | 30 | 55 | - | - | - | 57 700 | 1.33 |
| 52 | - | 84 | - | 56 | 18 | - | - | - | 52 400 | 1.35 |
| 53 | - | 105 | - | 10 | 52 | - | - | - | 60 000 | 1.35 |
| 54 | - | 102 | - | 42 | 18 | - | - | - | 58 900 | 1.37 |
| 55 | - | 111 | - | 25 | 18 | - | - | - | 60 100 | 1.40 |
| 56 | - | 131 | - | 10 | 18 | - | - | - | 67 400 | 1.43 |
| 57 | - | 129 | - | 108 | 31 | - | - | - | 84 100 | 1.51 |
| 58 | - | 170 | - | 144 | 40 | - | - | - | 111 200 | 1.61 |
| 59 | - | 221 | - | 191 | 50 | - | - | - | 144 700 | 1.68 |
| 60 | - | - | 58 | 102 | 78 | - | - | - | 91 200 | 1.71 |
| 61 | - | - | 62 | 143 | 21 | - | - | - | 93 100 | 1.68 |
| 62 | - | - | 59 | 105 | 93 | - | - | - | 95 000 | 1.82 |
| 63 | - | - | 60 | 106 | 18 | - | - | - | 85 400 | 1.68 |
| 64 | - | 75 | - | 66 | - | 19 | - | - | 49 400 | 1.46 |
| 65 | - | 67 | - | 37 | - | - | 39 | - | 44 300 | 1.40 |
| 66 | - | 61 | - | 56 | - | - | 20 | - | 41 600 | 1.43 |
| 67 | - | 75 | - | 42 | - | - | - | 42 | 52 300 | 1.61 |
| 68 | - | 75 | - | 68 | - | - | - | 18 | 51 400 | 1.56 |

[Example 69]

Production of poly[(benzyl acrylate)-co-(poly(ethylene glycol)methyl ether acrylate)-co-(acrylic acid)]

By treating poly[(benzyl acrylate)-co-(poly(ethylene glycol)methyl ether acrylate)-co-(1-ethoxyethyl acrylate)] obtained in Example 1 with 0.5N HCl at room temperature, an ethoxyethyl group was eliminated to obtain a terpolymer having a carboxyl group (1.176 g). A Z-average particle diameter and a polydispersity index of the obtained terpolymer in water were measured by dynamic light scattering (DLS), and a result of the Z-average particle diameter was 8.5 nm (polydispersity index: 0.14).

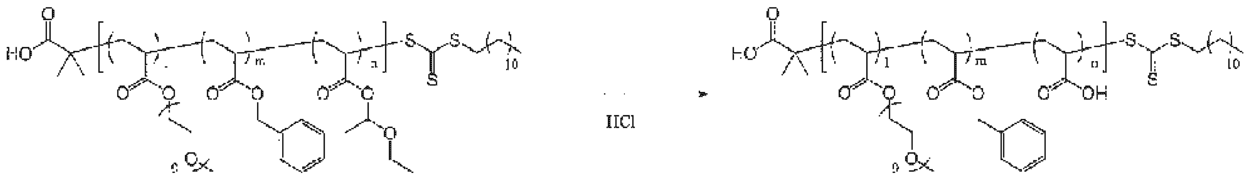


[Measurement apparatuses and conditions]

(1) DLS measurement

Apparatus: Zetasizer NanoZS/Malvern Instruments Ltd.
Measurement temperature: 25°C
Sample concentration: 10 mg/mL
Results: Fig. 3

[Example 70]

Method for producing (1,2-diaminocyclohexane)platinum(II)-encapsulating SCNP

In purified water (20 mL), a $Cl(H_2O)$ form ($DACHPt \cdot Cl \cdot H_2O$) of (1,2-diaminocyclohexane)platinum(II) (hereinafter, abbreviated as DACHPt) (65.28 mg) was dissolved and the mixture was stirred at 70°C for 2 hours. To this solution (5 mL), the terpolymer obtained in Example 69 (287.4 mg) was added, and the mixture was stirred at 50°C overnight. After completion of the stirring, the reaction solution was dialyzed and purified with the purified water as an external liquid to obtain a DACHPt-encapsulating SCNP (5 mL). Pt content of the DACHPt-encapsulating SCNP obtained after the purification was measured by inductively coupled plasma-atomic emission spectrometry (ICP-AES), and was 720 μg/mL (DACHPt: 1.14 mg/mL). Separately, the DACHPt-encapsulating SCNP (200 μL) was lyophilized, a solid content concentration thereof was calculated, then a ratio to the Pt content was obtained, a Pt binding amount per polymer was calculated, and a result thereof was 3.4 mol/mol. Further, a Z-average particle diameter and a polydispersity index of the obtained DACHPt-encapsulating SCNP was measured by dynamic light scattering (DLS), and a result of the Z-average particle diameter was 8.7 nm (polydispersity index: 0.14). The particle diameter of the SCNP before and after encapsulating the DACHPt is shown in Fig. 3. The particle diameter of the SCNP hardly varied before and after encapsulating the DACHPt. Results thereof are summarized in the following table.

[Measurement apparatuses and conditions]

(1) ICP-AES measurement

Apparatus: Sequential high frequency plasma light emitting apparatus ICPE-9000/SHIMADZU CORPORATION

Pretreatment apparatus: microwave sample pretreatment apparatus ETHOS EASY/Milestone General

Measurement wavelength: 214 nm

Standard solution: Platinum standard solution (Pt1000) for ICP analysis/FUJIFILM Wako Pure Chemical Corporation

(2) DLS measurement

Apparatus: Zetasizer NanoZS/Malvern Instruments Ltd.

Measurement temperature: 25°C

Sample concentration: 10 mg/mL

Results: Fig. 3

[Table 4]

| Example | Yield (mL) | Pt content (μg/mL) | Pt binding amount per polymer (mol/mol) | Z-average particle diameter (nm) | Polydispersity index |
|---|---|---|---|---|---|
| 70 | 5 | 720 | 3.4 | 8.7 | 0.14 |

[Example 71]

Production of $N_3$-poly[(benzyl acrylate)-co-(poly(ethylene glycol) methyl ether acrylate)-co-(1-ethoxyethyl acrylate)]

Synthesis was performed by using $N_3$-CTA in place of the chain transfer agent DDMAT used in Example 1. In toluene (17.3 mL), 2-(dodecylthiocarbonothioylthio)-2-methylpropionic acid 3-azido-1-propanol ester ($N_3$-CTA) (100 mg) was dissolved after being weighed to obtain an $N_3$-CTA/toluene stock solution ($N_3$-CTA concentration: 5.78 mg/mL). Similarly, 2,2'-azobis(2-methylpropionitrile) (AIBN) (10 mg) was weighed and dissolved in toluene (7.87 mL) to obtain an AIBN/toluene stock solution (AIBN concentration: 1.27 mg/mL). Separately, poly(ethylene glycol)methyl ether acrylate (mPEGA, average value (n) of the numbers of repetition times of ethylene glycol is 9) (2.592 g), benzyl acrylate (BnA) (0.700 g), 1-ethoxyethyl acrylate (0.156 g), the $N_3$-CTA/toluene stock solution (4.15 mL), and the AIBN/toluene stock solution (3.46 mL) were added, and polymerization was performed in an oil bath at 70°C. After a lapse of 90 minutes, the polymerization was stopped, and then a copolymer was recovered by subjecting the reaction solution to reprecipitation or dialysis against methanol. Since the obtained copolymer was basically a viscous form, in the reprecipitation, an operation of dropping the reaction solution into a centrifuge tube to which a poor solvent (hexane/ethyl acetate = 7/3 [v/v]) was added and recovering the solution by centrifugation (2 000 × g, 5 min) was repeated 3 times, and finally vacuum drying was performed to obtain $N_3$-poly[(benzyl acrylate)-co-(poly(ethylene glycol) methyl ether acrylate)-co-(1-ethoxyethyl acrylate)] (2.643 g). As a result of analyzing a polymerization degree of each of monomers and a number average molecular weight ($M_{n,NMR}$) of the obtained copolymer from a $^1$H-NMR spectrum measured by using NMR, the polymerization degree of mPEGA (n = 9) was 75, the polymerization degree of BnA was 55, the polymerization degree of EEA was 26, and $M_{n,NMR}$ was 48 900. Moreover, a molecular weight dispersion ($M_w/M_n$) of the obtained copolymer was measured by using the GPC and a result thereof was 1.28.

[Example 72]

Production of $N_3$-poly[(benzyl acrylate)-co-(poly(ethylene glycol)methyl ether acrylate)-co-(acrylic acid)]

By treating $N_3$-poly[(benzyl acrylate)-co-(poly(ethylene glycol) methyl ether acrylate)-co-(1-ethoxyethyl acrylate)] obtained in Example 71 with 0.5N HCl at room temperature, the ethoxyethyl group was eliminated to obtain $N_3$-poly [(benzyl acrylate)-co-(poly(ethylene glycol) methyl ether acrylate)-co-(acrylic acid)] having the carboxyl group (2.35 g). A Z-average particle diameter and a polydispersity index of the obtained copolymer in water were measured by dynamic light scattering (DLS), and a result of the Z-average particle diameter was 8.0 nm (polydispersity index: 0.13).

[Measurement apparatuses and conditions]

(1) $^1$H-NMR measurement

Apparatus: JNM-ECX400 (400 MHz)/JEOL Ltd.
Solvent: dimethyl sulfoxide-$d_6$ containing 0.03% tetramethylsilane/KANTO CHEMICAL CO., INC.
Sample concentration: 20 mg/mL
Measurement temperature: 25°C
Number of integration times: 256 times
Results: Fig. 4

(2) GPC measurement

Apparatus: HPLC-Prominence system/SHIMADZU CORPORATION
Detector: RID-10A Refractive index detector/SHIMADZU CORPORATION
Column: TSKgel α-2500 column/Tosoh Corporation
(Column size: 7.8 mm × 300 mm, particle size: 7 μm, and exclusion limit molecular weight: $5 \times 10^3$)
TSKgel α-4000 column/Tosoh Corporation
(Column size: 7.8 mm × 300 mm, particle size: 10 μm, and exclusion limit molecular weight: $4 \times 10^5$)
TSKgel guardcolum/Tosoh Corporation
Mobile phase: N,N-dimethyformamide (DMF) containing 10 mmol/L of lithium bromide
Temperature: 40°C
Flow rate: 0.5 mL/min
Sample concentration: 6 mg/mL
Standard substance: poly(methyl methacrylate) standard ReadyCal set, $M_p$ 800 - 2 200 000 Da/SIGMA
Results: Fig. 5

[Table 5]

| Example | Composition ratio (molar ratio to chain transfer agent) | | | | | Solvent | Temperature (°C) | Polymerization time (min) | Polymerization degree | | | $M_{n,NMR}$ | $M_w/M_n$ |
|---|---|---|---|---|---|---|---|---|---|---|---|---|---|
| | Chain transfer agent | Initiator | Monomer | | | | | | | | | | |
| | $N_3$-CTA | AIBN | mPEGA n=9 | BnA | EEA | | | | mPEGA n=9 | BnA | EEA | | |
| 72 | 1 | 0.5 | 100 | 70 | 30 | Toluene | 70 | 90 | 75 | 55 | 26 | 48 900 | 1.28 |

[Example 73]

Synthesis of a 1,4-diaminobutane-bonded copolymer

In DMF (47 mL), the copolymer obtained in Example 72 (2 350 mg) was dissolved after being weighed, COMU (1 111.1 mg) and TMP (527 μL) were added, and the mixture was stirred at room temperature for 3 hours. Then, N-(tert-butoxycarbonyl)-1,4-diaminobutane (2 512 μL) was added, and the mixture was stirred at 30°C for 3 days. The reaction solution was dialyzed and purified (dialysis membrane: Spectra/Por Regenerated Cellulose Membrane 6, molecular cut off: 3.5 kDa, and external liquid: methanol), and then the solvent was removed by the distillation under reduced pressure and the vacuum drying to recover the copolymer. An obtained N-Boc-1,4-diaminobutane-bonded copolymer was dissolved in a mixed solution of DCM and TFA [DCM/TFA = 5/3 (v/v)] (32 mL) and the mixture was stirred at room temperature overnight to perform deprotection, and then the solvent was removed by the distillation under reduced pressure and the vacuum drying to obtain the 1,4-diaminobutane-bonded copolymer (2 517 mg).

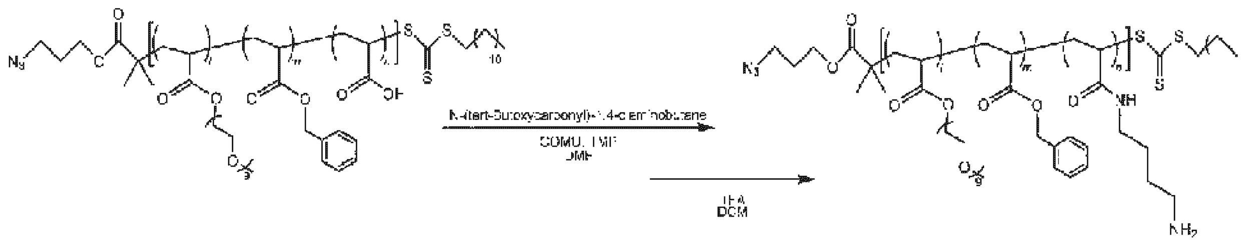


For the 1,4-diaminobutane-bonded copolymer, the number of 1,4-diaminobutane introduced to one molecule of the copolymer was analyzed from a $^1$H-NMR spectrum measured by using NMR, and a result thereof was 26 mol/mol.

[Measurement apparatuses and conditions]

(1) $^1$H-NMR measurement

Apparatus: JNM-ECX400 (400 MHz)/JEOL Ltd.
Solvent: dimethyl sulfoxide-$d_6$ containing 0.03% tetramethylsilane/KANTO CHEMICAL CO., INC.
Sample concentration: 20 mg/mL
Measurement temperature: 25°C
Number of integration times: 256 times
Results: Fig. 6

[Table 6]

| Example | Copolymer used | Number of 1,4-diaminobutane introduced to one molecule of copolymer (mol/mol) |
|---|---|---|
| 73 | Example 72 | 26 |

[Example 74]

Production of DM1-SPDP-1,4-diaminobutane-bonded copolymer

To DCM (1 mL), mertansine (DM1) (81 mg) and 2,5-dioxopyrrolidin-1-yl 3-(pyridin-2-yldisulfaneyl)propanoate (SPDP) (29 mg) were added, dissolved, N,N-diisopropylethylamine (DIPEA) (16 μL) was added, and the mixture was stirred at 30°C for 3 hours. In DCM (4 mL), the copolymer obtained in Example 73 (200 mg) was dissolved, added to the reaction solution, and the obtained reaction liquid was stirred at 30°C for 24 hours. The reaction solution was dialyzed and purified (dialysis membrane: Spectra/Por Regenerated Cellulose Membrane 6, molecular cut off: 3.5 kDa, and external liquid: methanol), then the solvent was removed by the distillation under reduced pressure and the vacuum drying, and the copolymer was recovered to obtain the DM1-SPDP-1,4-diaminobutane-bonded copolymer (189 mg). A Z-average particle diameter and a polydispersity index of the obtained copolymer in water were measured by dynamic light scattering (DLS), and a result of the Z-average particle diameter was 13 nm (polydispersity index: 0.23).

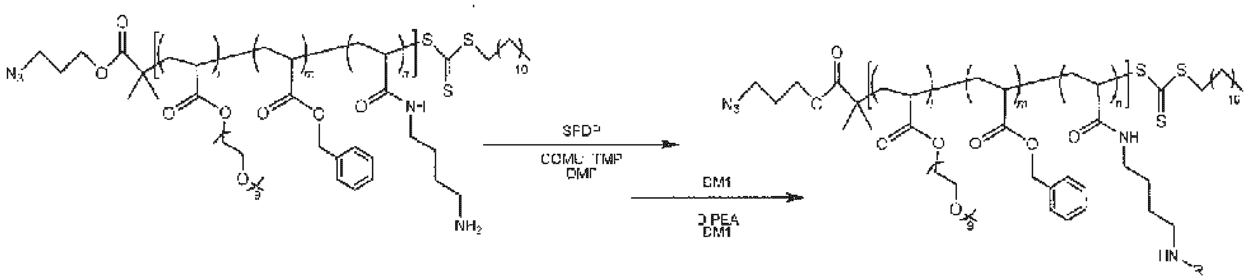


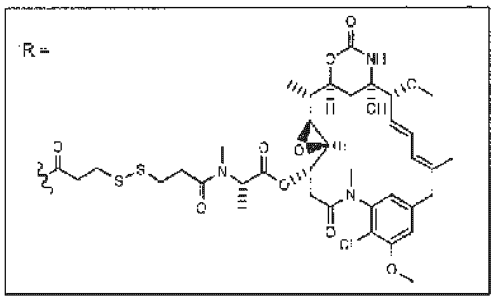

For the DM1-SPDP-1,4-diaminobutane-bonded copolymer, the number of DM1 introduced to one molecule of the copolymer was analyzed from a $^1$H-NMR spectrum measured by using NMR, and a result thereof was 26 mol/mol.

[Measurement apparatuses and conditions]

(1) $^1$H-NMR measurement

Apparatus: JNM-ECX400 (400 MHz)/JEOL Ltd.

Solvent: dimethyl sulfoxide-$d_6$ containing 0.03% tetramethylsilane/KANTO CHEMICAL CO., INC.
Sample concentration: 20 mg/mL
Measurement temperature: 25°C
Number of integration times: 256 times
Results: Fig. 7

[Table 7]

| Example | Copolymer used | Number of DM1 introduced to one molecule of copolymer (mol/mol) |
|---|---|---|
| 74 | Example 73 | 26 |

[Example 75]

Production of anti-HER2 affibody micelle drug complex

The copolymer obtained in Example 74 and the anti-HER2 affibody were complexed. To a glass vial into which an anti-Her2 affibody (6 × His tag-containing Z(HER2:2891), amino acid SEQ ID (9)) stock solution (0.53 mg/mL) (14 mL) was dispensed, 0.5 mM tris(2-carboxyethyl)phosphine (TCEP) dissolved in PBS (2 μL) was added, and the mixture was stirred at 37°C for 15 minutes. Then, DBCO-PEG4-maleimide (1.4 mg) was dissolved in DMAc (N,N-dimethylacetamide) (1 mL) and added, and the mixture was stirred at 37°C for 15 minutes to react with a terminal cysteine residue of the affibody. To that, an aqueous solution (10 mg/mL) of L-cysteine (168149, Sigma-Aldrich) in PBS (20 μL) was added, and the mixture was stirred at 37°C for 1 minute to stop the reaction. The reaction liquid was recovered, dialyzed against purified water overnight (Slide-A-Lyzer™ Dialysis Cassette, molecular cut off: 3.5 K, 66110, Thermo Fisher Scientific), and then subjected to ultrafiltration (Vivaspin Turbo 15, molecular cut off: 3 K) to remove an unreacted modifying reagent. Dilution was performed in a measuring flask to exactly 5 mL with PBS, and the solution (1 mL) was dispensed into glass vials. Next, the copolymer obtained in Example 74 (60 mg) was added and the mixture was left to stand in BICELL™ at -20°C for 1 day. Then, the solution obtained by thawing at 4°C was recovered. In anion exchange chromatography, a copolymer-bonded affibody and a single copolymer (raw material) or a free affibody (raw material) were separated depending on the retention time, washed by ultrafiltration (Vivaspin Turbo 15, molecular cut off: 10K) with PBS, and then concentrated. Next, immobilized metal ion affinity chromatography (IMAC) targeting a His tag protein expressed in the 6 × His tag-containing Z(HER2:2891) affibody was performed to obtain the anti-HER2 affibody micelle drug complex as an object. A Z-average particle diameter and a polydispersity index of the obtained anti-HER2 affibody micelle drug complex in water were measured by dynamic light scattering (DLS), and a result of the Z-average particle diameter was 11 nm (polydispersity index: 0.17).

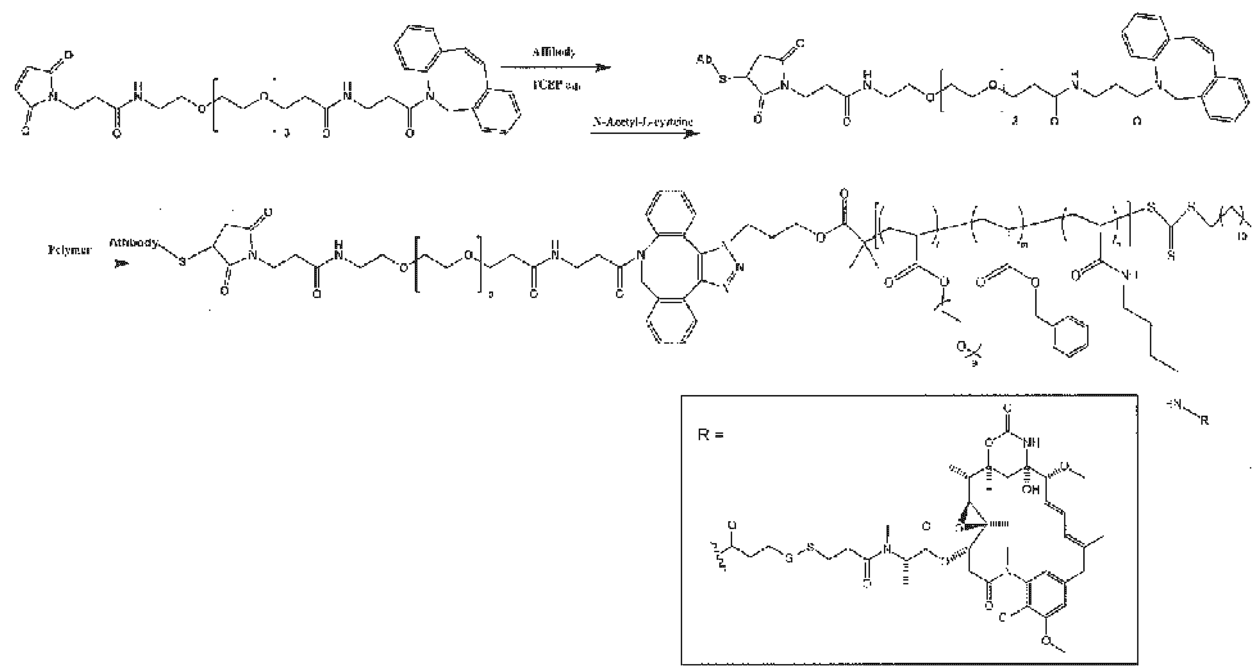

[Measurement apparatuses and conditions]

(1) Anion exchange column purification

Apparatus: AKTA primePlus/Cytiva
Detector: UV/280 nm
Column: HiTrap Q XL (1 mL)/Cytiva
Mobile phase A: 20 mmol/L Tris buffer (pH 10)
Mobile phase B: 20 mmol/L Tris buffer (pH 10) + 1M NaCl (pH 10)
Temperature: 25°C
Flow rate: 1.0 mL/min
Sample loop: 0.5 mL

(2) IMAC Purification

Apparatus: AKTA primePlus/Cytiva
Detector: UV/280 nm
Column: HisTrap HP (5 mL)/Cytiva
Mobile phase A: 20 mmol/L trisodium phosphate buffer + 150 mmol/L NaCl (pH 7.6)
Mobile phase B: 20 mmol/L trisodium phosphate buffer + 500 mmol/L NaCl + 500 mmol/L imidazole (pH 7.6)
Temperature: 25°C
Flow rate: 2.0 mL/min
Sample loop: 0.5 mL

(3) DLS measurement

Apparatus: Zetasizer NanoZS/Malvern Instruments Ltd.
Measurement temperature: 25°C
Sample concentration: 10 mg/mL
Results: Fig. 8

[Example 76]

Production of anti-EGFR affibody micelle drug complex

The copolymer obtained in Example 74 and the anti-EGFR affibody (6 × His tag-containing Z(EGFR:03115), amino acid SEQ ID (10)) were complexed in a similar manner as in Example 75. A Z-average particle diameter and a polydispersity index of the obtained anti-EGFR affibody micelle drug complex in water were measured by dynamic light scattering, and a result of the Z-average particle diameter was 14 nm (polydispersity index: 0.19).

[Measurement apparatuses and conditions]

(1) Anion exchange column purification

Apparatus: AKTA primePlus/Cytiva
Detector: UV/280 nm
Column: HiTrap Q XL (1 mL)/Cytiva
Mobile phase A: 20 mmol/L Tris buffer (pH 10)
Mobile phase B: 20 mmol/L Tris buffer (pH 10) + 1M NaCl (pH 10)
Temperature: 25°C
Flow rate: 1.0 mL/min
Sample loop: 0.5 mL

(2) IMAC Purification

Apparatus: AKTA primePlus/Cytiva
Detector: UV/280 nm
Column: HisTrap HP5mL)/Cytiva
Mobile phase A: 20 mmol/L trisodium phosphate buffer + 150 mmol/L NaCl (pH 7.6)
Mobile phase B: 20 mmol/L trisodium phosphate buffer + 500 mmol/L NaCl + 500 mmol/L imidazole (pH 7.6)
Temperature: 25°C
Flow rate: 2.0 mL/min
Sample loop: 0.5 mL

(3) DLS measurement

Apparatus: Zetasizer NanoZS/Malvern Instruments Ltd.
Measurement temperature: 25°C
Sample concentration: 10 mg/mL
Results: Fig. 9

[Example 77]

Production of anti-HER3 affibody micelle drug complex

The copolymer obtained in Example 74 and the anti-HER3 affibody (3 × HE tag-containing Z(HER3:Z08699), amino acid SEQ ID (11)) were complexed in a similar manner as in Example 75. A Z-average particle diameter and a polydispersity index of the obtained anti-HER3 affibody micelle drug complex in water were measured by dynamic light scattering, and a result of the Z-average particle diameter was 11 nm (polydispersity index: 0.15).

[Measurement apparatuses and conditions]

(1) Anion exchange column purification

Apparatus: AKTA primePlus/Cytiva
Detector: UV/280 nm
Column: HiTrap Q XL (1 mL)/Cytiva
Mobile phase A: 20 mmol/L Tris buffer (pH 10)
Mobile phase B: 20 mmol/L Tris buffer (pH 10) + 1M NaCl (pH 10)
Temperature: 25°C
Flow rate: 1.0 mL/min
Sample loop: 0.5 mL

(2) IMAC Purification

Apparatus: AKTA primePlus/Cytiva
Detector: UV/280 nm
Column: HisTrap HP (5 mL)/Cytiva
Mobile phase A: 20 mmol/L trisodium phosphate buffer + 150 mmol/L NaCl (pH 7.6)
Mobile phase B: 20 mmol/L trisodium phosphate buffer + 500 mmol/L NaCl + 500 mmol/L imidazole (pH 7.6)
Temperature: 25°C
Flow rate: 2.0 mL/min
Sample loop: 0.5 mL

(3) DLS measurement

Apparatus: Zetasizer NanoZS/Malvern Instruments Ltd.

Measurement temperature: 25°C
Sample concentration: 10 mg/mL
Results: Fig. 10

[Comparative Example 1]

Preparation of an oxaliplatin solution

To a 5.9 (w/v)% glucose solution (5.58 mL) was added an ELPLAT I.V. infusion solution 50 mg (Yakult Honsha Co., Ltd.) (1 mL) to prepare a 5 (w/v)% glucose solution containing oxaliplatin (760 μg).

[Comparative Example 2]

Preparation of DM1 solution

The mertansine (DM1) (Cayman CHEMICAL) was dissolved in dimethyl sulfoxide (DMSO: FUJIFILM Wako Pure Chemical Corporation) to prepare DM1 solutions of 60 μmol/L, 20 μmol/L, 6.7 μmol/L, 2.2 μmol/L, 0.74 μmol/L, 0.25 μmol/L, 0.082 μmol/L, and 0.027 μmol/L. Next, after a dilution series of 60 nmol/L, 20 nmol/L, 6.7 nmol/L, 2.2 nmol/L, 0.74 nmol/L, 0.25 nmol/L, 0.082 nmol/L, and 0.027 nmol/L were created by respectively diluting these solutions with RPMI1640 (hereinafter, RPMI1640 medium) containing 10% fetal bovine serum (Merck KGaA), these solutions were respectively added to a cell culture microplate in equal amounts to a cell culture liquid, and diluted to 1/2, thereby evaluating the cytotoxic effects thereof at a final DM1 concentration of 30 nmol/L, 10 nmol/L, 3.3 nmol/L, 1.1 nmol/L, 0.37 nmol/L, 0.12 nmol/L, 0.041 nmol/L, or 0.014 nmol/L.

[Comparative Example 3]

Preparation of 6 × His tag-containing Z(HER2:2891) solution

After a dilution series of 600 ng/mL, 200 ng/mL, 60 ng/mL, 20 ng/mL, 6.0 ng/mL, 2.0 ng/mL, 0.60 ng/mL, 0.20 ng/mL, and 0.060 ng/mL was created by diluting a 6 × His tag-containing Z(HER2:2891), which is the anti-HER2 affibody (autologous preparation, physiological saline solution at an anti-HER2 affibody concentration of 0.393 mg/mL, amino acid SEQ ID (9)) with the RPMI1640 medium, these solutions were respectively added to the cell culture microplate in an equal amount to the cell culture liquid, and diluted to 1/2, thereby evaluating the cytotoxic effects thereof at a final anti-HER2 affibody concentration of 300 ng/mL, 100 ng/mL, 30 ng/mL, 10 ng/mL, 3.0 ng/mL, 1.0 ng/mL, 0.30 ng/mL, 0.10 ng/mL, or 0.030 ng/mL.

[Comparative Example 4]

Preparation of 6 × His tag-containing Z(EGFR:03115) solution

After a dilution series of 600 ng/mL, 200 ng/mL, 60 ng/mL, 20 ng/mL, 6.0 ng/mL, 2.0 ng/mL, 0.60 ng/mL, 0.20 ng/mL, and 0.060 ng/mL was created by diluting a 6 × His tag-containing Z(EGFR:03115), which is the anti-EGFR affibody (autologous preparation, physiological saline solution at an anti-EGFR affibody concentration of 0.101 mg/mL, amino acid SEQ ID (10)) with the RPMI1640 medium, these solutions were respectively added to the cell culture microplate in an equal amount to the cell culture liquid, and diluted to 1/2, thereby evaluating the cytotoxic effects thereof at a final anti-EGFR affibody concentration of 300 ng/mL, 100 ng/mL, 30 ng/mL, 10 ng/mL, 3.0 ng/mL, 1.0 ng/mL, 0.30 ng/mL, 0.10 ng/mL, or 0.030 ng/mL.

[Test Example 1] Drug efficacy test

Cancer-carrying models obtained by subcutaneously transplanting a mouse colon cancer cell line C26 (American Type Culture Collection) into female nude mice (BALB/c-nu/nu, 7 weeks old; Charles River Laboratories Japan, Inc.) were used for the drug efficacy test.

The mouse colon cancer cell line C26 subcultured in a $CO_2$ incubator was suspended in a liquid medium (Dulbecco's Modified Eagle's Medium-high glucose, Sigma-Aldrich), and injected subcutaneously in the back of each of the nude mice such that the number of cells per mouse was $1 \times 10^6$/100 μL. Then, the nude mice were raised for about 1 week, and then administration of the agent was started when an average value of tumor volumes was grown to about 30 $mm^3$. The DACHPt-encapsulating SCNP (DACHPt-encapsulating SCNP prepared using the copolymer of Example 70)

was administered through the tail veins of the mice (3 times every other day), and antitumor effects were evaluated based on the tumor volumes (4 to 5 mice per 1 group). As a comparison, an oxaliplatin solution (Comparative Example 1) was used, and the oxaliplatin solution was administered in the same manner. The dose of each preparation was 8 mg/kg (3.9 mg/kg in terms of Pt) as the maximum administrable dose for the oxaliplatin solution, and 3 mg/kg in terms of Pt for the DACHPt-encapsulating SCNP.

A change in the tumor volume over time is shown in Fig. 11. For the DACHPt-encapsulating SCNP, T/C was 0.4 after 14 days from administration [T/C: ratio of the tumor volume of the drug administration group (T) to that of the control group (C)]. For the oxaliplatin solution (Comparative Example 1), T/C was 1.1 after 14 days from administration. In addition, after 14 days from administration, it was confirmed that the DACHPt-encapsulating SCNP significantly suppressed growth of a tumor as compared with the control (Student's t-test). The above results indicate that the DACHPt-encapsulating SCNP has an excellent antitumor effect as compared with the oxaliplatin solution.

[Test Example 2] Cytotoxic test

A HER2 antigen-positive human gastric cancer cell line HCC-1954 (American Type Culture Collection) or a HER2 antigen-negative human breast cancer cell line MDA-MB-468 (American Type Culture Collection) was adjusted to a cell concentration of $2.0 \times 10^4$ cells/mL with the RPMI1640 medium, and 100 μL each was added to the 96 well microplate for cell culture and cultured overnight. On the next day, the evaluation specimen diluted with the RPMI1640 medium (100 μL) was separately added to the microplate, and cultured for 4 days at 37°C under 5% $CO_2$. After the culturing, the CellTiter-Glo™ luminescent cell viability assay (Promega Corporation) was added to the microplate, and the mixture was stirred and left to stand at room temperature for 10 minutes. The light emission amount was then measured with the plate reader (Molecular Devices, LLC.). The cell viability was calculated by the following equation.

$$\text{Cell viability (\%)} = a/b \times 100$$

In the equation, a represents an average value (n = 3) of light emission amounts of sample wells, and b represents an average value (n = 3) of light emission amounts of sample non-added wells.

An $IC_{50}$ value, which is an agent concentration at which the cell viability is 50%, was calculated using SAS™ 9.4 Software (SAS Institute Japan Ltd.).

[Table 8]

| Example | $IC_{50}$(nmol/L) | |
|---|---|---|
| | HER2-positive HCC-1954 | HER2-negative MDA-MB-468 |
| Comparative example 2 | 0.11 | 0.41 |
| Comparative example 3 | >38 | >38 |
| Example 75 | 0.30 | 4.9 |

An EGFR antigen-positive human breast cancer cell line MDA-MB-468 (American Type Culture Collection) and an EGFR antigen-negative human breast cancer cell line MDA-MB-453 (RIKEN BRC CELL BANK) were adjusted to a cell concentration of $2.0 \times 10^4$ cells/mL and $4.0 \times 10^4$ cells/mL with the RPMI1640 medium, respectively, and 100 μL each was added to a 96 well microplate for cell culture and cultured overnight. On the next day, the evaluation specimen diluted with the RPMI1640 medium (100 μL) was separately added to the microplate, and the cells were cultured for 4 days at 37°C under 5% $CO_2$. After the culturing, CellTiter-Glo™ luminescent cell viability assay (Promega Corporation) was added to the microplate, and the mixture was stirred and left to stand at room temperature for 10 minutes. The light emission amount was then measured with a plate reader (Molecular Devices, LLC.). The cell viability and the $IC_{50}$ value were calculated.

[Table 9]

| Example | $IC_{50}$(nmol/L) | |
|---|---|---|
| | EGFR-positive MDA-MB-468 | EGFR-negative MDA-MB-453 |
| Comparative example 4 | >38 | >38 |
| Example 76 | 3.2 | >3.8 |

## Claims

1. A copolymer in which a target-specific molecule is bonded to a copolymer X comprising structural units represented by the following formulas (A), (B), and (C):

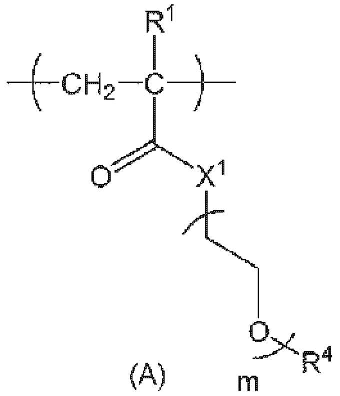

(A) ,

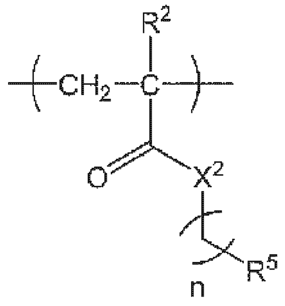

(B) ,

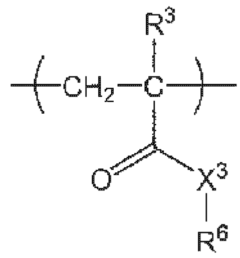

(C)

wherein, $R^1$, $R^2$, and $R^3$ are the same or different and represent a hydrogen atom or a $C_{1-3}$ alkyl group, $R^4$ represents a $C_{1-3}$ alkyl group, $R^5$ represents a hydrogen atom, a $C_{1-18}$ alkyl group, a 3- to 8- membered cycloalkyl group optionally having a substituent, an adamantyl group, a $C_{6-18}$ aryl group optionally having a substituent, or a 5-to 10- membered heteroaryl group optionally having a substituent, $X^1$, $X^2$, and $X^3$ are the same or different and represent an oxygen atom, a sulfur atom, or N-$R^7$, $R^6$ represents a hydrogen atom, a leaving group, or a linker, $R^7$ represents a hydrogen atom or a $C_{1-3}$ alkyl group, m represents an integer of 1 to 100, and n represents an integer of 0 to 3.

2. The copolymer according to claim 1, wherein the polymer X is a copolymer formed by polymerization of three types of monomers represented by the following general formulas (1) to (3):

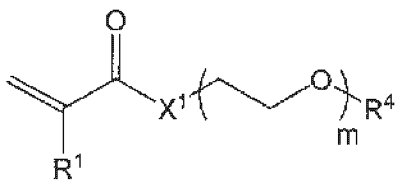

(1) ,

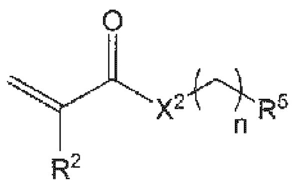

(2) ,

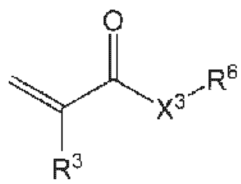

(3)

wherein, $R^1$, $R^2$, and $R^3$ are the same or different and represent a hydrogen atom or a $C_{1-3}$ alkyl group, $R^4$ represents a $C_{1-3}$ alkyl group, $R^5$ represents a hydrogen atom, a $C_{1-18}$ alkyl group, a 3- to 8-membered cycloalkyl group optionally having a substituent, an adamantyl group, a $C_{6-18}$ aryl group optionally having a substituent, or a 5-to 10-membered heteroaryl group optionally having a substituent, $X^1$, $X^2$, and $X^3$ are the same or different and represent an oxygen atom, a sulfur atom, or N-$R^7$, $R^6$ represents a hydrogen atom, a leaving group, or a linker, $R^7$ represents a hydrogen atom or a $C_{1-3}$ alkyl group, m represents an integer of 1 to 100, and n represents an integer of 0 to 3.

3. The copolymer according to claim 1 or 2, wherein $R^1$ is a hydrogen atom.

4. The copolymer according to claim 1 or 2, wherein $R^2$ is a hydrogen atom.

5. The copolymer according to claim 1 or 2, wherein $R^3$ is a hydrogen atom.

6. The copolymer according to claim 1 or 2, wherein $R^4$ is a methyl group.

7. The copolymer according to claim 1 or 2, wherein $R^5$ is a $C_{6-18}$ aryl group optionally having a substituent.

8. The copolymer according to claim 1 or 2, wherein $R^5$ is a phenyl group.

9. The copolymer according to claim 1 or 2, wherein $R^6$ is a hydrogen atom.

**10.** The copolymer according to claim 1 or 2, wherein the leaving group of $R^6$ is a group represented by the following formula (4).

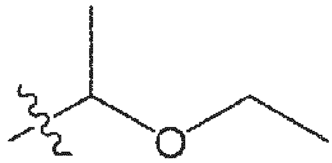

(4)

**11.** The copolymer according to claim 1 or 2, wherein the linker of $R^6$ is a group represented by the following formula (5):

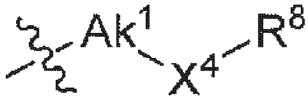

(5)

wherein, $R^8$ represents a hydrogen atom or a drug, $Ak^1$ represents a $C_{1-7}$ alkylene bond, and $X^4$ represents an oxygen atom, a sulfur atom, or $-N(R^7)-$, in which $R^7$ represents a hydrogen atom or a $C_{1-3}$ alkyl group.

**12.** The copolymer according to claim 1 or 2, wherein $X^1$ is an oxygen atom.

**13.** The copolymer according to claim 1 or 2, wherein $X^2$ is an oxygen atom.

**14.** The copolymer according to claim 1 or 2, wherein $X^3$ is an oxygen atom or NH.

**15.** The copolymer according to claim 1 or 2, wherein m is an integer of 4 to 22.

**16.** The copolymer according to claim 1 or 2, wherein n is 1.

**17.** The copolymer according to claim 1, wherein a ratio of structural units (A), (B), and (C) is composed of 0.01 to 100 parts by mass of (B) and 0.1 to 100 parts by mass of (C) with respect to 1 part by mass of (A).

**18.** The copolymer according to claim 2, wherein 0.01 to 100 parts by mass of monomer (2) and 0.1 to 100 parts by mass of monomer (3) are polymerized with respect to 1 part by mass of monomer (1).

**19.** The copolymer according to claim 1 or 2, wherein the copolymer has a number average molecular weight of 5 000 to 150 000.

**20.** The copolymer according to claim 1 or 2, wherein the target-specific molecule is an affibody.

**21.** The copolymer according to claim 20, wherein the affibody is an anti-HER2 affibody, an anti-HER3 affibody, an anti-EGFR affibody, an anti-PD-L1 affibody, an anti-TNF$\alpha$ affibody, an anti-fibrinogen affibody, an anti-transferrin affibody, an anti-HSA affibody, an anti-insulin affibody, an anti-IgG affibody, an anti-IgM affibody, an anti-IgA affibody, or an anti-IgE affibody.

**22.** The copolymer according to claim 20, wherein the affibody is a molecule including the following amino acid sequences (2) to (6).

AEAKYAKEMRNAYWEIALLPNLTNQQKRAFIRKLYDDPSQSSELLSEAKKLNDSQAPK (SEQ ID (2)) (2)

AEAKYAKEKYNAYYEIWQLPNLTKYQKAAFIGKLQDDPSQSSELLSEAKKLNDSQAPK (SEQ ID (3)) (3)

VDNKFNKEMWAAWEEIRNLPNLNGWQMTAFIASLVDDPSQSANLLAEAKKLNDAQAPK (SEQ ID (4)) (4)
VDNKFNKEMWIAWEEIRDLPNLNGWQMTAFIASLLDDPSQSANLLAEAKKLNDAQAPK (SEQ ID (5)) (5)
AEAKYAKEMWIAWEEIRNLPNLNGWQMTAFIAKLLDDPSQSSELLSEAKKLNDSQAPK (SEQ ID (6)) (6)
AEAKYAKERNAAAYEILYLPNLTNAQKWAFIWKLDDDPSQSSELLSEAKKLNDSQAPK (SEQ ID (7)) (7)
AEAKYAKERNKAAYEILYLPNLTNAQKWAFIWKLDDDPSQSSELLSEAKKLNDSQAPK (SEQ ID (8)) (8)

**23.** A drug complex comprising the copolymer according to claim 1 or 2 and a drug.

**24.** The drug complex according to claim 23, wherein the drug is an antimetabolite, an alkylating agent, an anthracycline, an antibiotic, a mitotic inhibitor, a topoisomerase inhibitor, a proteasome inhibitor, or an anti-hormone agent.

**25.** The drug complex according to claim 23, wherein the drug is DM0, DM1, DM2, DM3, DM4, emtansine, auristatin E, auristatin phenylalanine phenylenediamine (AFP), monomethyl auristatin E, monomethyl auristatin D, monomethyl auristatin F, paclitaxel, docetaxel, irinotecan, topotecan, nogitecan, amsacrine, etoposide, teniposide, mizantrone, SN-38, exatecan, or deruxtecan.

**26.** The drug complex according to claim 23, wherein the bond of the target-specific molecule or the drug to the copolymer X is a covalent bond or a non-covalent bond.

**27.** A single chain nanoparticle comprising the copolymer according to claim 1 or 2.

**28.** A single chain nanoparticle comprising the drug complex according to claim 23.

**29.** A pharmaceutical composition comprising the copolymer according to claim 1 or 2.

**30.** A pharmaceutical composition comprising the drug complex according to claim 23.

FIG. 1

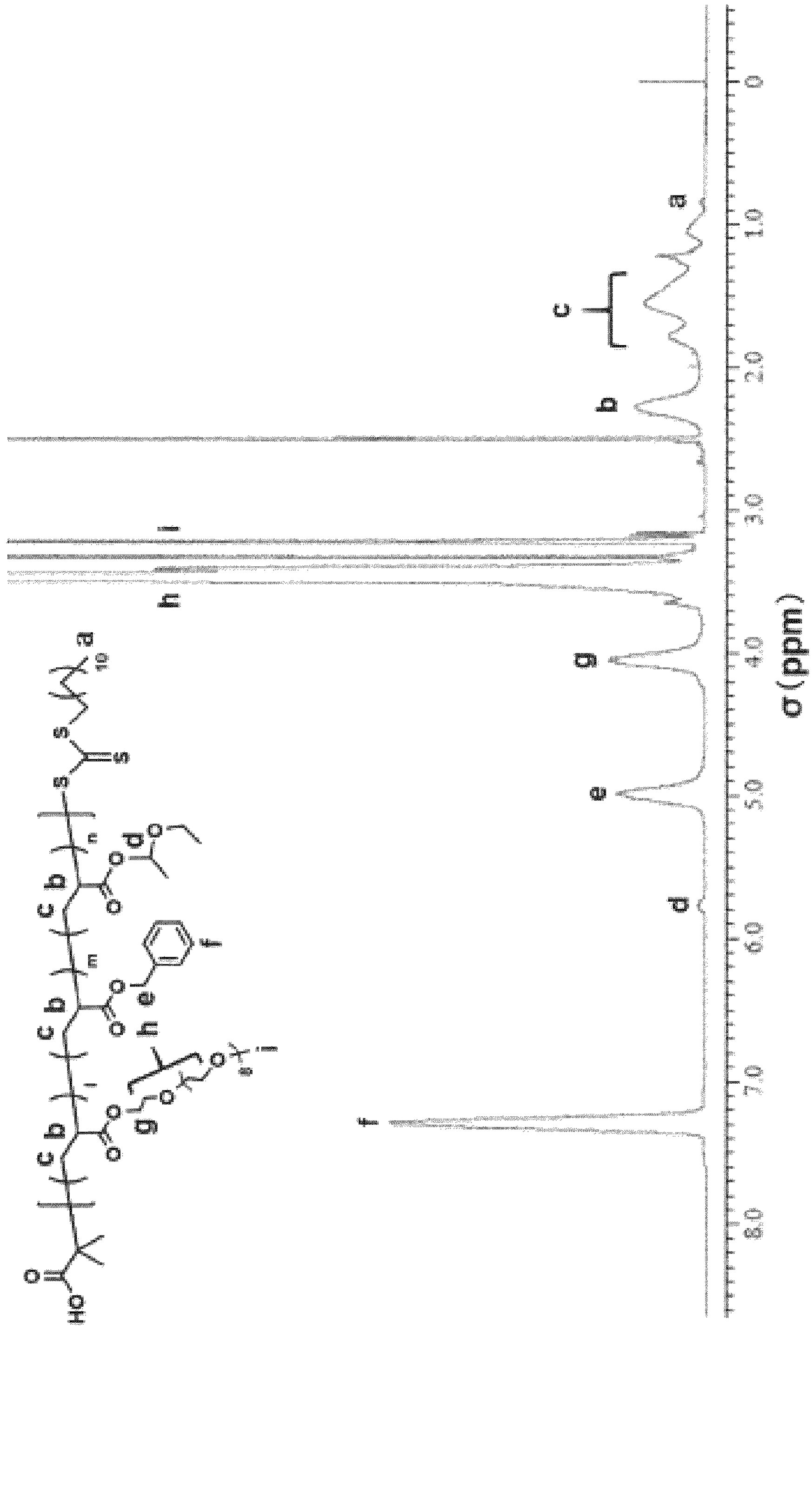

FIG. 2

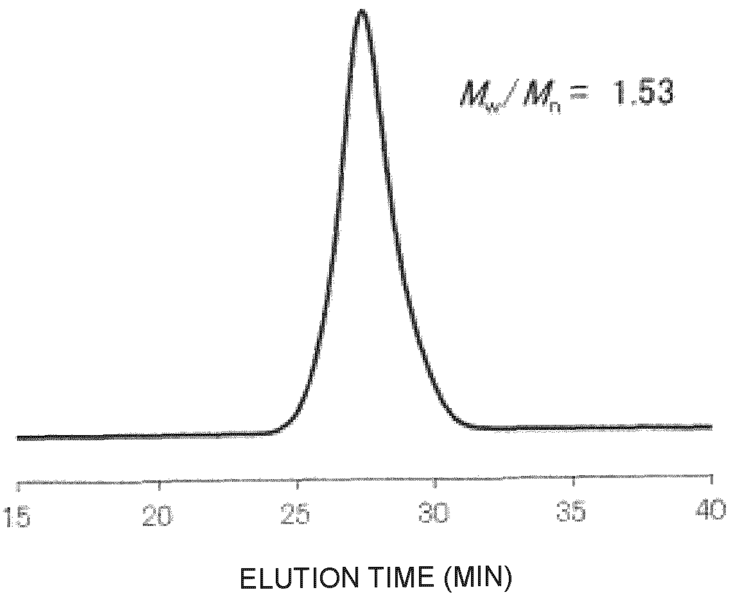
$M_w / M_n$ = 1.53
15
20
25
30
35
40
ELUTION TIME (MIN)

FIG. 3

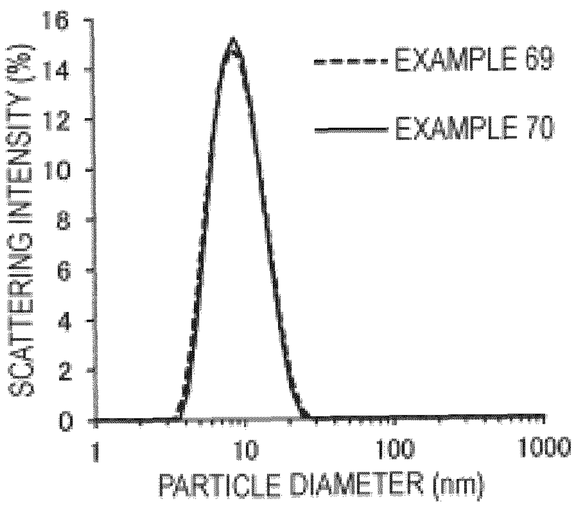
16
14
12
10
8
6
4
2
0
SCATTERING INTENSITY (%)
EXAMPLE 69
EXAMPLE 70
1
10
100
1000
PARTICLE DIAMETER (nm)

FIG. 4

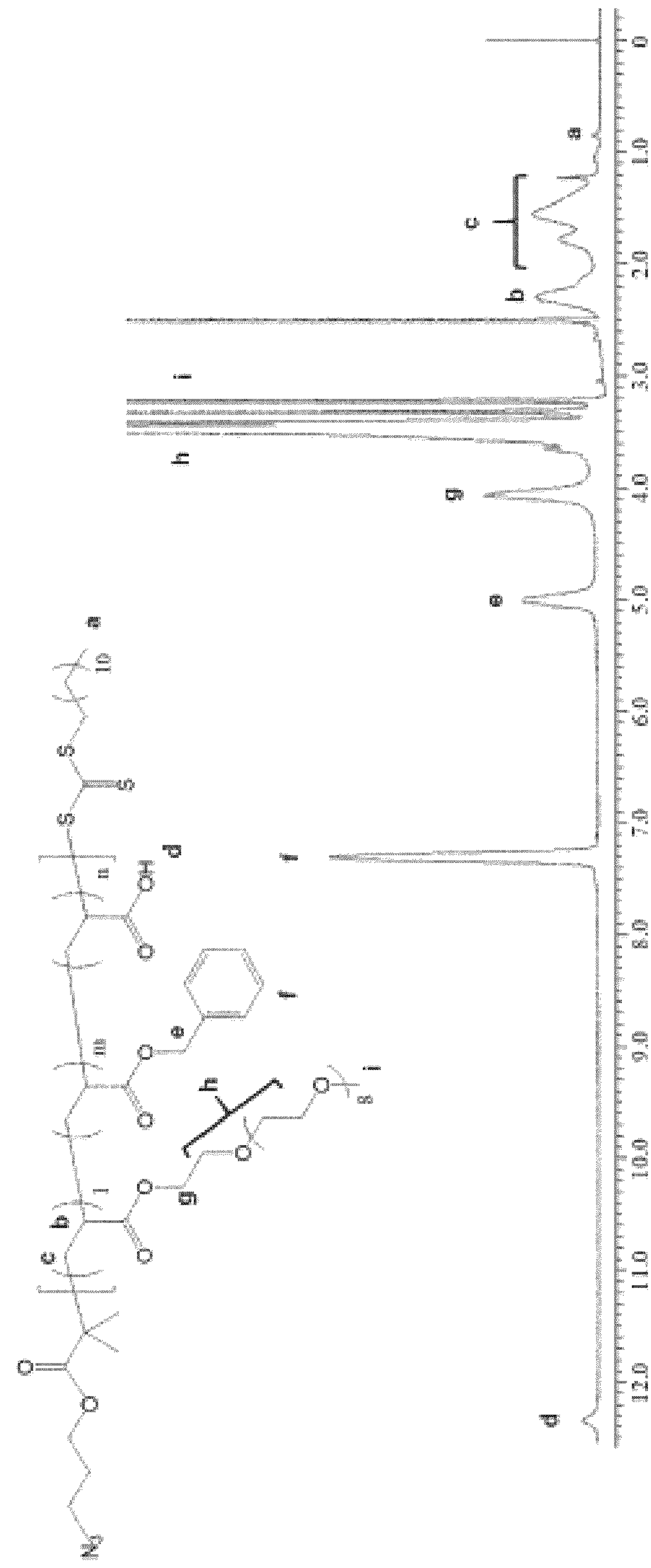

FIG. 5

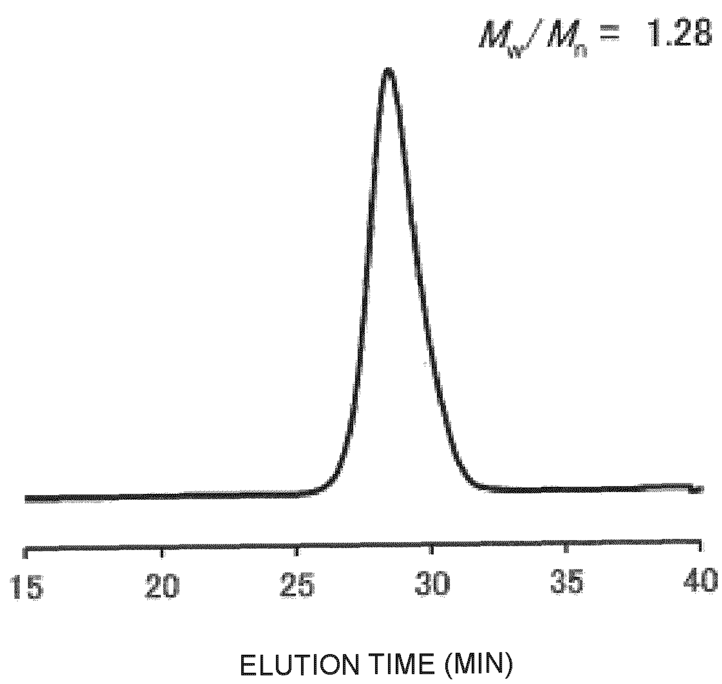

*FIG. 6*

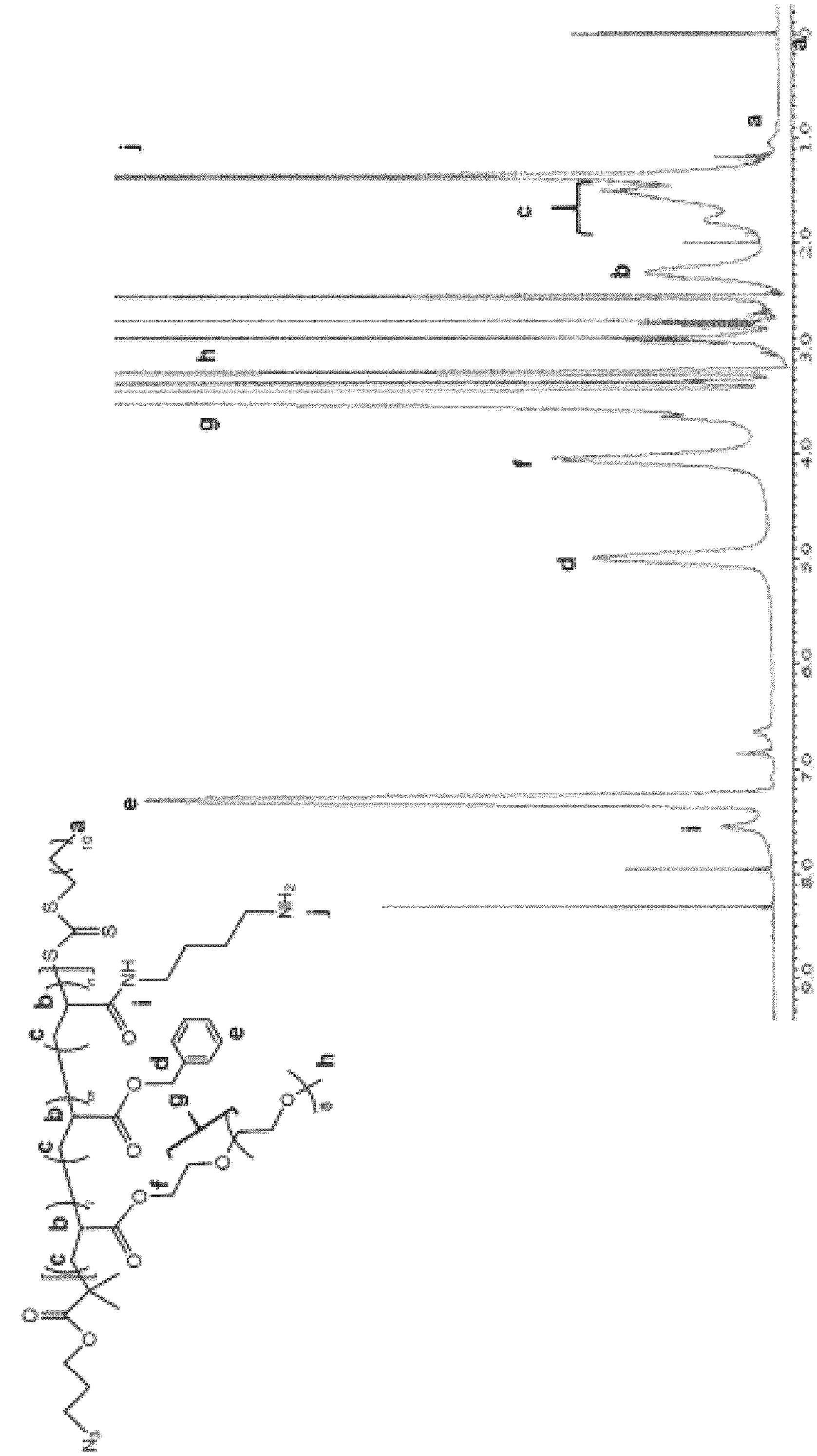

FIG. 7

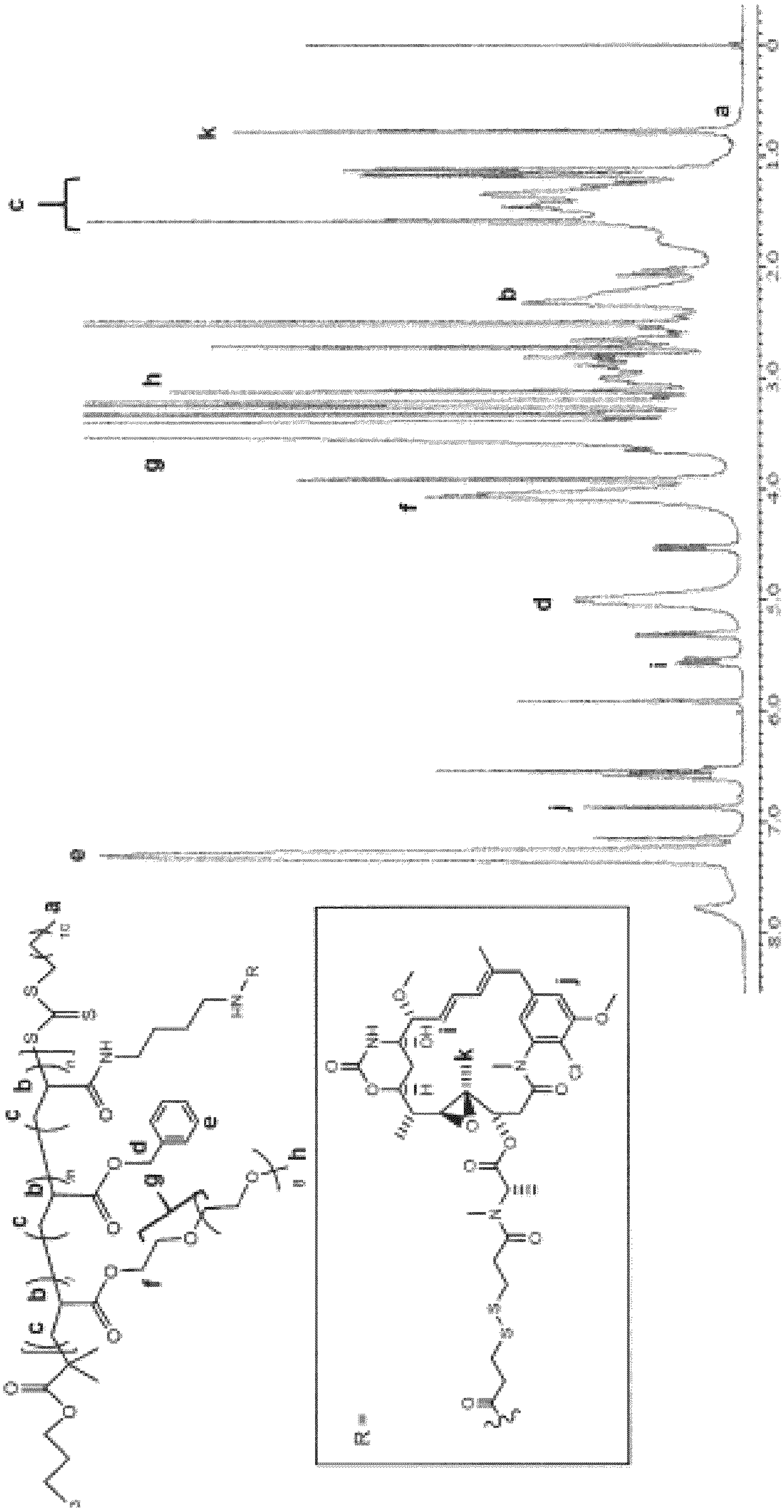

FIG. 8

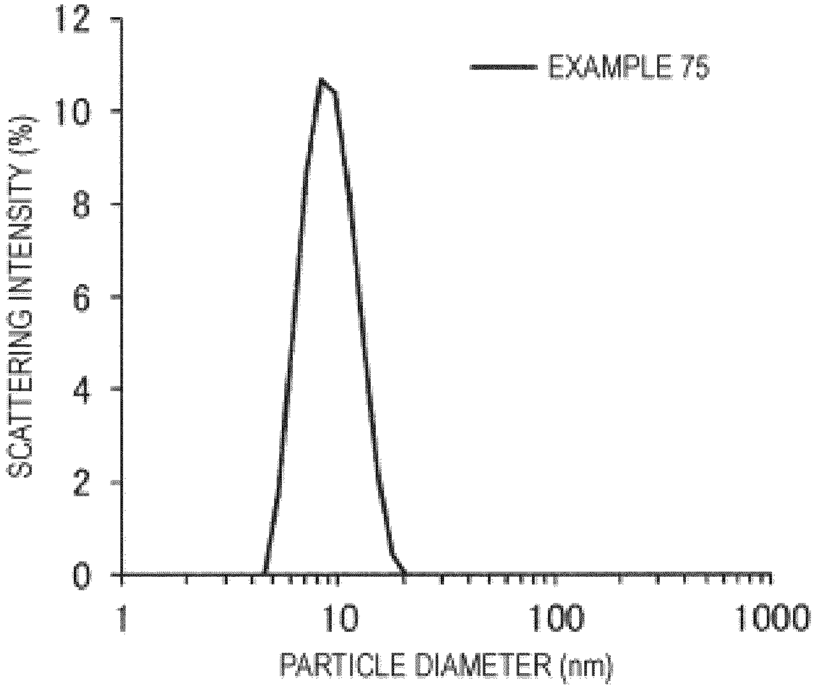
12
10
8
6
4
2
0
SCATTERING INTENSITY (%)
EXAMPLE 75
1
10
100
1000
PARTICLE DIAMETER (nm)

FIG. 9

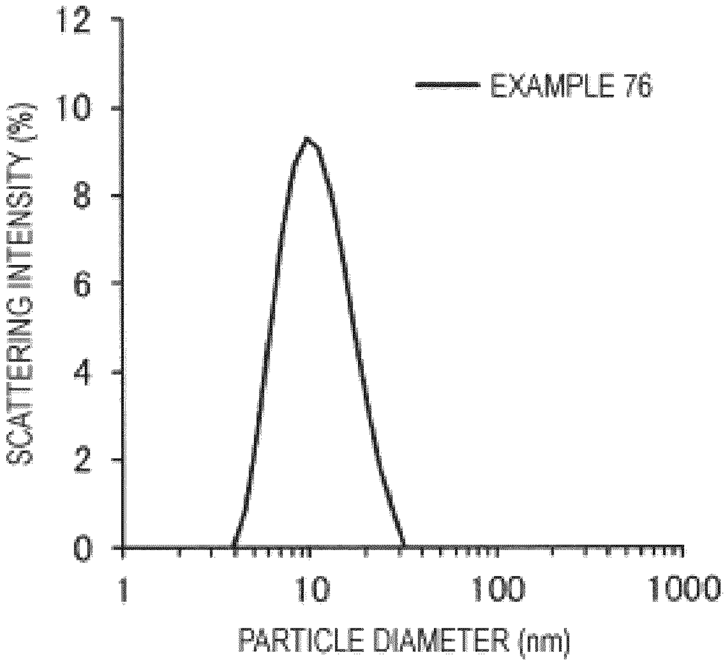
12
10
8
6
4
2
0
SCATTERING INTENSITY (%)
EXAMPLE 76
1
10
100
1000
PARTICLE DIAMETER (nm)

FIG. 10

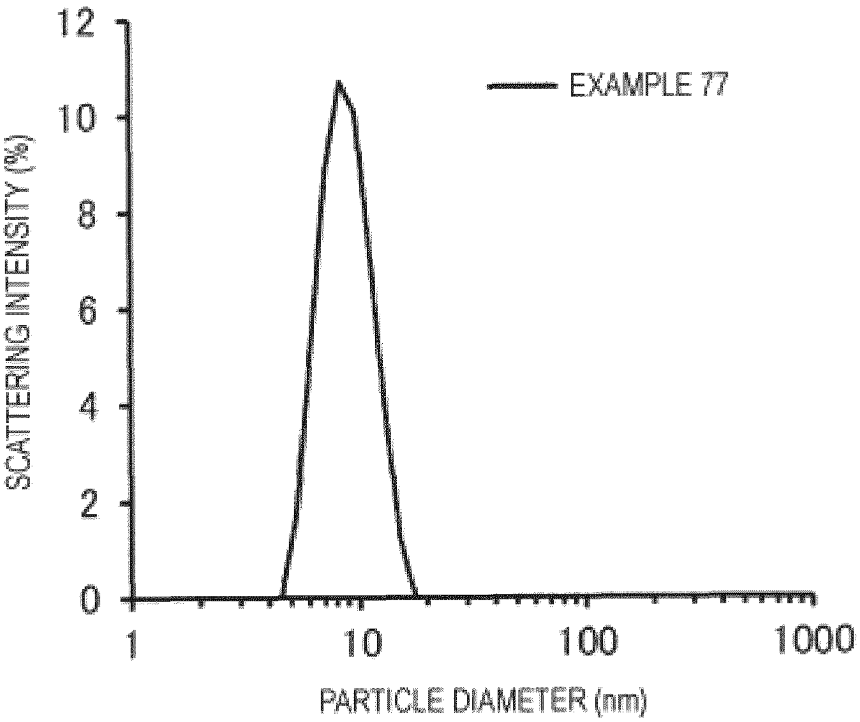
EXAMPLE 77
12
10
8
6
4
2
0
SCATTERING INTENSITY (%)
1
10
100
1000
PARTICLE DIAMETER (nm)

*FIG. 11*

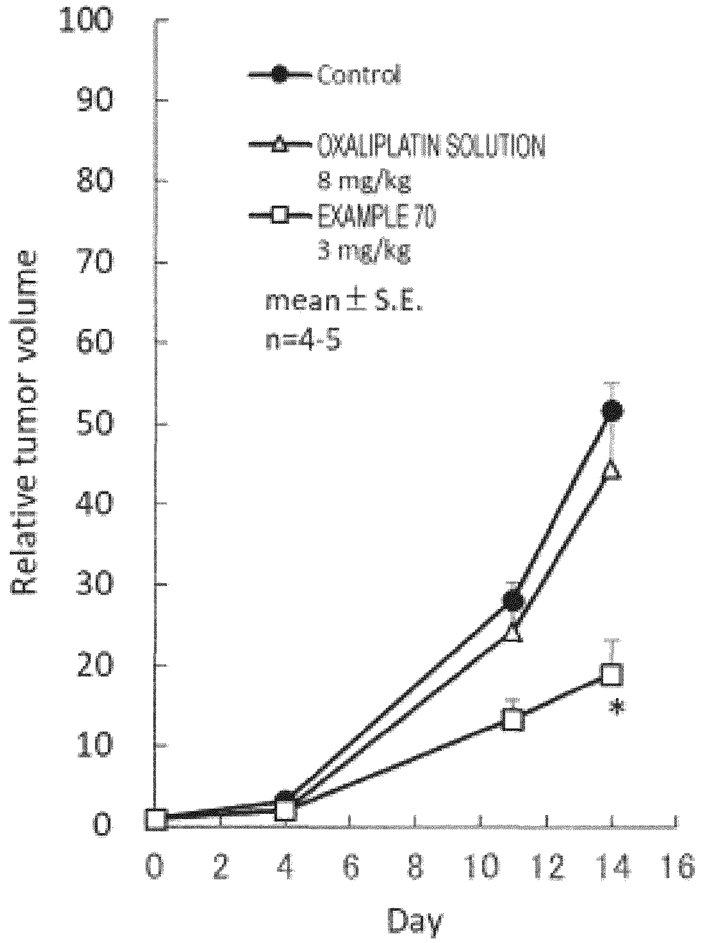


*; P < 0.05 (student's t-test) vs. Control

## INTERNATIONAL SEARCH REPORT

International application No.
**PCT/JP2023/029357**

**A. CLASSIFICATION OF SUBJECT MATTER**

***C08F 220/10***(2006.01)i; ***A61K 31/537***(2006.01)i; ***A61K 39/395***(2006.01)i; ***A61K 45/00***(2006.01)i; ***A61K 47/58***(2017.01)i; ***A61K 47/64***(2017.01)i; ***A61P 35/00***(2006.01)i; ***C08F 220/30***(2006.01)i
FI: C08F220/10; A61K31/537; A61K39/395 Y; A61K45/00; A61K47/58; A61K47/64; A61P35/00; C08F220/30

According to International Patent Classification (IPC) or to both national classification and IPC

**B. FIELDS SEARCHED**

Minimum documentation searched (classification system followed by classification symbols)

C08F220/10; A61K31/537; A61K39/395; A61K45/00; A61K47/58; A61K47/64; A61P35/00; C08F220/30

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

JSTPlus/JMEDPlus/JST7580 (JDreamIII); CAplus/REGISTRY (STN)

**C. DOCUMENTS CONSIDERED TO BE RELEVANT**

| Category* | Citation of document, with indication, where appropriate, of the relevant passages | Relevant to claim No. |
|---|---|---|
| X | US 2017/0233714 A1 (DUKE UNIVERSITY) 17 August 2017 (2017-08-17) claims, paragraphs [0003], [0008], [0010]-[0012], [0014]-[0032], [0043], [0076]-[0094], [0097], [0098], [0108]-[0111], [0131], [0135]-[0137], [0165], [0166], [0168], [0172], [0174], [0177] | 1-7, 9, 12-19, 23-24, 26-30 |
| A | WO 2008/035785 A1 (JSR CORP.) 27 March 2008 (2008-03-27) entire text | 1-30 |
| A | JP 2012-108460 A (FUJIFILM CORP.) 07 June 2012 (2012-06-07) entire text | 1-30 |
| A | JP 2016-122174 A (DNP FINE CHEMICALS CO., LTD.) 07 July 2016 (2016-07-07) entire text | 1-30 |
| A | JP 2017-105722 A (TOYO INK SC HOLDINGS CO., LTD.) 15 June 2017 (2017-06-15) entire text | 1-30 |
| P, A | WO 2022/168967 A1 (KOWA COMPANY, LTD.) 11 August 2022 (2022-08-11) entire text | 1-30 |

☐ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **04 October 2023** | **24 October 2023** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | Telephone No. |

Form PCT/ISA/210 (second sheet) (January 2015)

**INTERNATIONAL SEARCH REPORT**

International application No.
**PCT/JP2023/029357**

**Box No. I Nucleotide and/or amino acid sequence(s) (Continuation of item 1.c of the first sheet)**

1. With regard to any nucleotide and/or amino acid sequence disclosed in the international application, the international search was carried out on the basis of a sequence listing:
   a. ☑ forming part of the international application as filed:
      - ☑ in the form of an Annex C/ST.25 text file.
      - ☐ on paper or in the form of an image file.
   
   b. ☐ furnished together with the international application under PCT Rule 13*ter*.1(a) for the purposes of international search only in the form of an Annex C/ST.25 text file.
   
   c. ☐ furnished subsequent to the international filing date for the purposes of international search only:
      - ☐ in the form of an Annex C/ST.25 text file (Rule 13*ter*.1(a)).
      - ☐ on paper or in the form of an image file (Rule 13*ter*.1(b) and Administrative Instructions, Section 713).

2. ☐ In addition, in the case that more than one version or copy of a sequence listing has been filed or furnished, the required statements that the information in the subsequent or additional copies is identical to that forming part of the application as filed or does not go beyond the application as filed, as appropriate, were furnished.

3. Additional comments:

   "In the form of an Annex C/ST.25 text file" above should be understood as "in ST.26 format".

Form PCT/ISA/210 (continuation of first sheet) (January 2015)

**INTERNATIONAL SEARCH REPORT**
**Information on patent family members**

International application No.
**PCT/JP2023/029357**

| Patent document cited in search report | Publication date (day/month/year) | Patent family member(s) | Publication date (day/month/year) |
|---|---|---|---|
| US 2017/0233714 A1 | 17 August 2017 | WO 2017/112825 A2<br>EP 3393500 A2<br>CN 109152818 A | |
| WO 2008/035785 A1 | 27 March 2008 | KR 10-2009-0071607 A | |
| JP 2012-108460 A | 07 June 2012 | (Family: none) | |
| JP 2016-122174 A | 07 July 2016 | WO 2016/024596 A1<br>CN 106574125 A | |
| JP 2017-105722 A | 15 June 2017 | (Family: none) | |
| WO 2022/168967 A1 | 11 August 2022 | (Family: none) | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 3270592 B
- US 5831012 A
- WO 2009016043 A
- WO 2009080810 A
- WO 2011056124 A
- WO 2014053586 A
- WO 2007065635 A
- WO 2017072280 A


### Non-patent literature cited in the description


- **H. CABRAL et al.** *Nat. Nanotechnol.*, 2011, vol. 6, 815-823
- **JOSE A. POMPOSO**. *Single-Chain Polymer Nanoparticles: Synthesis, Characterization, Simulations, and Applications*, 2017
- **CHARI, R.V. et al.** *Angew.Chem.Int.Ed.*, 2014, vol. 53, 3796-3827
- **JAGADEESH, D.** ; **SMITH, M.R.** *Curr.Treat.Options Oncol.*, 2016, vol. 17, 55
- **DUCRY, L.** ; **STUMP, B.** *Bioconjugate Chem.*, 2010, vol. 21, 5-13
- **CASI, G.** ; **NERI, D.** *J.Controlled Release*, 2012, vol. 161, 422-428
- **WU, A. M.** ; **SENTER, P.D.** *Nat.Biotechnol.*, 2005, vol. 23, 1137-1146
- **NORD, K. et al.** *Nature Biotechnol.*, 1997, vol. 15, 772-777
- **XU, TIANQI et al.** *Cancers*, 2020, vol. 13 (1), 85